# EUROPÄISCHE PATENTANMELDUNG

(11) **EP 4 806 474 A1**
(43) Veröffentlichungstag der Anmeldung: **16.09.2026**
(21) Anmeldenummer: 25162915.0
(22) Anmeldetag: 11.03.2025
(51) Int. Cl.: A61M 1/00, A61M 3/02

(54) **MODULARES WUNDSPÜLSYSTEM MIT ABSAUGUNG**

(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Die Erfindung betrifft eine Vorrichtung zur Abgabe einer medizinischen Flüssigkeit an einen Patienten, aufweisend einen elastisch verformbaren Behälter zur Aufnahme einer Flüssigkeit, einen Adapter, der mit dem Behälter fluidleitend verbindbar ist, ein Abgabeelement zur Abgabe einer Flüssigkeit aus der Vorrichtung, und ein Absaugelement zur Abführung einer Flüssigkeit, wobei die Vorrichtung ausgebildet und eingerichtet ist, eine Flüssigkeit durch Zusammendrücken des Behälters mit einem Druck zu beaufschlagen, und hierdurch die Flüssigkeit aus der Vorrichtung an einen Patienten abzugeben, und gleichzeitig durch das Absaugelement die abgegebene Flüssigkeit von dem Patienten abzuführen.

## Beschreibung

### GEBIET DER ERFINDUNG

Die vorliegende Erfindung betrifft das Gebiet der Medizintechnik, insbesondere Vorrichtungen und Verfahren zur Anwendung in der chirurgischen Reinigung infizierten Gewebes. Gegenstand der Erfindung ist insbesondere eine chirurgische Reinigungsvorrichtung, die zur mechanischen Reinigung und Spülung von infizierten Gelenkendoprothesen im Rahmen von DAIR-Prozeduren verwendbar sein kann.

### TECHNISCHER HINTERGRUND

In der septischen Knochenchirurgie ist die Reinigung von debridierten Knochen- und Weichgewebearealen bei der Revision von infizierten Gelenkendoprothesen und die Reinigung von infizierten Osteosyntheseplatten durch Lavagierung bekannt. Dafür werden medizinische Spülvorrichtungen, sogenannte Lavage-Systeme, eingesetzt. Diese Lavage-Systeme werden hierin auch als "Lavagevorrichtung" bezeichnet. Einige dieser Lavage-Systeme werden als "Jet-Lavage-Systeme" beschrieben. Diese Systeme erzeugen einen Flüssigkeitsstrahl, der die Abtragung infizierten Gewebes unterstützt. Als Reinigungsflüssigkeiten sind physiologische Kochsalzlösung, Ringer-Lösung und Ringer-Lactat-Lösung bekannt. Das Ziel der Lavagierung besteht in der Entfernung von Resten des infizierten Gewebes, von Biofilmresten, von Blut und von Wundsekret. Lavage-Systeme sind beispielhaft in den Patentschriften EP3187208A1, US4583531A, US5779702A, US6059754A und US2019151531A1 beschrieben.

In der septischen Chirurgie gewinnen die sogenannten DAIR-Prozeduren (Debridement, antibiotics and implant retention) zur Behandlung von infizierten Kniegelenkendoprothesen, Hüftgelenk Endoprothesen und Schultergelenkendoprothesen zunehmend an Bedeutung. Ein Ziel dieser OP-Technik ist es, die Infektion der Gelenkendoprothese und des umgebenden Gewebes nach Eröffnung des Gelenks durch Debridement und anschließende lokale Applikation von Antibiotika so zu behandeln, dass das künstliche Gelenk erhalten bleiben kann. Die kostenintensive und für den Patienten belastende Implantation einer Revisionsgelenkendoprothese soll hierbei vermieden werden.

Diese Behandlungsverfahren werden insbesondere bei früh infizierten künstlichen Gelenkendoprothesen angewendet, Dabei werden zuerst bewegliche Implantatkomponenten, wie z.B. PE-Inlays, aus dem künstlichen Gelenk entfernt. Infiziertes Gewebe wird abgetragen ("debridiert") und Biofilme, die an den Metalloberflächen der Gelenkendoprothese anhaften, versucht man durch Bürsten und Lavagieren möglichst vollständig zu entfernen. Danach werden neue bewegliche Gelenkkomponenten, wie PE-Inlays, in die gereinigte Gelenkendoprothese eingesetzt. Problematisch ist dabei die Entfernung der Biofilme an Metalloberflächen, insbesondere an den Kondylen (Gelenkfortsätzen) von Kniegelenken. Biofilme können eine hohe Adhäsion zu Oberflächen aufweisen. Hierdurch können trotz Bürsten und anschließendem Lavagieren Biofilme oder Biofilmreste an Implantatoberflächen verbleiben. Diese Biofilmreste enthalten lebende Mikroorganismen und können zu einer Reinfektion der Gelenkendoprothese und des umgebenden Gewebes führen.

Zur Behandlung von Biofilmen können antimikrobielle Zusammensetzungen verwendet werden. WO2017027418A1 beschreibt eine antimikrobielle Zusammensetzung, die eine Glycosidase enthält. Weitere antimikrobielle Zusammensetzungen sind in US9668989B2, WO9300100A2, WO2024118545A1, WO2022081737A1, WO2017027418A1 und WO2014179754A2 beschrieben.

EP4458343A1 beschreibt eine Vorrichtung zur Herstellung einer Wundspüllösung, die eine wirkstoffhaltige Spritze und einen Adapter zum Anschluss an einen Behälter mit einer Flüssigkeit umfasst. Hierbei kann die Flüssigkeit aus dem Behälter entnommen werden, innerhalb der Spritze mit dem Wirkstoff gemischt werden, und in den Behälter zurückgegeben werden.

Wundspüllösungen können mit Hilfe einer Spritzflasche oder anderen Lavage-Systemen verabreicht werden.

Wundspülsysteme mit einer komprimierbaren Flasche sind in US6468253B1, EP1113829B1, US8021346B2, US7662125B2, US9629953B2 und EP3117849B1 beschrieben.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Eine Aufgabe der Erfindung ist es, Biofilme durch eine Reinigungsflüssigkeit von einer Implantatoberfläche zu entfernen. Dadurch können die klebrigen Biofilme nach der Ablösung nicht wieder auf der Implantatoberfläche anhaften. Eine weitere Aufgabe der Erfindung ist es, die mit der Reinigungsflüssigkeit vermischten Biofilmreste möglichst schnell aus dem Gelenksitus zu entfernen, so dass eine Ablagerung der abgelösten Biofilmreste auf dem umgebenden Gewebe, insbesondere der Gelenkkapsel, wirksam verhindert werden kann.

Eine weitere Aufgabe ist die Bereitstellung eines modularen Spülsystems, das eine wechselbare Düse aufweist. Hierdurch kann, beispielsweise während einer DAIR-Prozedur, eine mikrobiell kontaminierte Düse gegen eine saubere, bevorzugt sterile, Düse ausgewechselt werden, wobei der übrige Teil der Vorrichtung weiterverwendet werden kann.

Eine weitere Aufgabe der Erfindung ist die Bereitstellung eines modularen Spülsystems aus mehreren Teilen, welches bei einer Druckbeaufschlagung der Spülflüssigkeit eine stabile fluidleitende Verbindung innerhalb des Systems gewährleistet.

Eine weitere Aufgabe besteht in der Entwicklung einer Spülvorrichtung, die es erlaubt, Geschwüre und ähnliche Wunden an der Körperoberfläche so zu reinigen, dass eine Kontamination des umliegenden Körperoberfläche und der Umgebung des Patienten durch mikrobiell kontaminierte Spülflüssigkeit vermindert oder weitgehend vermieden wird.

Die Vorrichtung ist bevorzugt möglichst einfach und kostengünstig aus Kunststoffteilen zu fertigen. Weiterhin kann die Vorrichtung bevorzugt durch Autoklavierung, oder Elektronenstrahl- oder Gammasterilisation sterilisierbar sein.

Einige oder alle Aufgaben werden gelöst durch hierin beschriebene Verfahren und Vorrichtungen, insbesondere denjenigen, die in den Patentansprüchen beschrieben sind. Bevorzugte Ausführungsformen der Erfindung werden nachstehend beschrieben.

Eine erste Ausführungsform beschreibt eine Vorrichtung zur Abgabe einer medizinischen Flüssigkeit an einen Patienten, aufweisend
einen elastisch verformbaren Behälter zur Aufnahme einer Flüssigkeit,
einen Adapter, der mit dem Behälter fluidleitend verbindbar ist,
ein Abgabeelement zur Abgabe einer Flüssigkeit aus der Vorrichtung,
und ein Absaugelement zur Abführung einer Flüssigkeit,
wobei die Vorrichtung ausgebildet und eingerichtet ist, eine Flüssigkeit durch Zusammendrücken des Behälters mit einem Druck zu beaufschlagen, und hierdurch die Flüssigkeit aus der Vorrichtung an einen Patienten abzugeben, und gleichzeitig durch das Absaugelement die abgegebene Flüssigkeit von dem Patienten abzuführen.

Eine zweite Ausführungsform beschreibt eine Vorrichtung gemäß der ersten Ausführungsform, wobei der Behälter, der Adapter und das Abgabeelement jeweils als miteinander lösbar verbindbare Teile ausgebildet sind.

Eine dritte Ausführungsform beschreibt eine Vorrichtung gemäß der ersten oder zweiten Ausführungsform, wobei das Absaugelement einen Schlauch oder ein Rohr aufweist.

Eine vierte Ausführungsform beschreibt eine Vorrichtung nach einer der vorangehenden Ausführungsformen, wobei das Absaugelement durch eine Schelle gehalten ist, wobei die Schelle bevorzugt drehbar gelagert an der Vorrichtung angeordnet ist.

Eine fünfte Ausführungsform beschreibt eine Vorrichtung nach einer der vorangehenden Ausführungsformen, wobei das Absaugelement einen flexiblen oder gekrümmten Leitungsbereich aufweist.

Eine sechste Ausführungsform beschreibt eine Vorrichtung nach einer der vorangehenden Ausführungsformen, wobei das Absaugelement ein Gelenk aufweist, wobei das Gelenk bevorzugt als Scharnier oder Kugelgelenk ausgebildet ist.

Eine siebte Ausführungsform beschreibt eine Vorrichtung gemäß der sechsten Ausführungsform, wobei die Vorrichtung ausgebildet und eingerichtet ist, eine Flüssigkeit an einer ersten Position abzugeben, und die abgegebene Flüssigkeit an einer zweiten Position aufzunehmen, wobei die zweite Position relativ zu der ersten Position veränderlich ist, ohne die gesamte Vorrichtung zu bewegen.

Eine achte Ausführungsform beschreibt eine Vorrichtung nach einer der vorangehenden Ausführungsformen, wobei der Behälter ein erstes Verbindungselement aufweist,
wobei der Adapter ein zweites Verbindungselement, ein drittes Verbindungselement, und eine erste Durchführung zur Abgabe einer Flüssigkeit aus dem Behälter aufweist,
wobei das Abgabeelement ein viertes Verbindungselement und eine zweite Durchführung zur Abgabe einer Flüssigkeit aus der Vorrichtung aufweist,
und wobei die Vorrichtung ausgebildet und eingerichtet ist, durch Eingreifen des ersten Verbindungselements in das zweite Verbindungselement und durch Eingreifen des dritten Verbindungselements in das vierte Verbindungselement eine druckfeste fluidleitende Verbindung zwischen dem Behälter, dem Adapter und dem Abgabeelement herzustellen.

Eine neunte Ausführungsform beschreibt eine Vorrichtung nach einer der vorangehenden Ausführungsformen, weiterhin aufweisend ein Septum, das den Behälter flüssigkeitsdicht verschließt, wobei der Adapter eine erste Durchführung zur Abgabe einer Flüssigkeit aus dem Behälter aufweist, und wobei der Adapter ausgebildet und eingerichtet ist, durch Inkontaktbringen des Adapters mit dem Behälter das Septum zu durchdringen, um eine fluidleitende Verbindung zwischen dem Behälter und der ersten Durchführung herzustellen.

Eine zehnte Ausführungsform beschreibt eine Vorrichtung nach einer der vorangehenden Ausführungsformen, wobei das Abgabeelement weiterhin ein fünftes Verbindungselement aufweist, welches unabhängig von dem dritten Verbindungselement und dem vierten Verbindungselement mit einem Zubehörteil verbindbar ist.

Eine elfte Ausführungsform beschreibt eine Vorrichtung gemäß der zehnten Ausführungsformen, wobei das Zubehörteil ausgewählt ist aus der Gruppe bestehend aus einem Schlauch, einem Spritzschutzschirm und einer Reinigungsbürste.

Eine zwölfte Ausführungsform beschreibt eine Vorrichtung nach einer der vorangehenden Ausführungsformen, wobei der Behälter mit einer sterilen Flüssigkeit befüllt ist, wobei die Flüssigkeit bevorzugt einen antiseptischen Wirkstoff umfasst, der ausgewählt ist aus der Gruppe bestehend aus Chlorhexidinglukonat, Benzalkoniumchlorid, Octenidindihydrochlorid, einem Alkalihypochlorit, einem Erdalkalihypochlorit, einem Alkalichlorit, und/oder wobei die Flüssigkeit Natriumdodecylsulfat oder physiologische Kochsalzlösung umfasst.

Eine dreizehnte Ausführungsform beschreibt eine Vorrichtung gemäß der zwölften Ausführungsform, wobei die sterile Flüssigkeit eine wässrige Lösung umfasst, die ausgewählt ist aus der Gruppe bestehend aus
einer Lösung von Benzalkoniumchlorid mit einer Benzalkoniumchlorid-Konzentration von 50 bis 1500 ppm;
einer Lösung von Chlorhexidindiglukonat und Benzalkoniumchlorid, wobei die Konzentration an Chlorhexidindiglukonat 50 bis 600 ppm beträgt und die Konzentration an Benzalkoniumchlorid 50 bis 250 ppm beträgt;
einer Lösung von Octenidindihydrochlorid mit einer Konzentration von 50 bis 2000 ppm;
einer Lösung eines Alkalihypochlorits mit einer Konzentration an Hypochlorit von 50 bis 2000 ppm;
einer Lösung eines Erdalkalihypochlorits mit einer Konzentration an Hypochlorit 50 bis 2000 ppm;
einer Lösung eines Alkalichlorits mit einer Konzentration an Chlorit von 50 bis 2000 ppm,
einer Lösung von Natriumdodecylsulfat mit einer Konzentration an Natriumdodecylsulfat von 50 bis 2000 ppm; und
einer physiologischen Kochsalzlösung mit einer Konzentration an Natriumchlorid von 0,9 Gew.-%;
wobei die ppm-Angaben sich jeweils auf einen Masse/Masse-Anteil im Verhältnis der jeweils genannten gelösten Stoffe zur Gesamtmasse der wässrigen Lösung beziehen.

Eine vierzehnte Ausführungsform beschreibt eine Vorrichtung nach einer der vorangehenden Ausführungsformen, wobei das Abgabeelement eine Austrittsöffnung aufweist, und das Absaugelement eine Absaugöffnung aufweist, wobei die Austrittsöffnung eine Querschnittsfläche aufweist, die kleiner oder gleich groß als/wie die Querschnittsfläche der Absaugöffnung ist.

Eine fünfzehnte Ausführungsform beschreibt eine Vorrichtung gemäß einer der vorangehenden Ausführungsformen, wobei die Vorrichtung als in einem versiegelten, sterilen Packmittel angeordneter Teilesatz ausgebildet ist, und den Behälter, den Adapter und das Abgabeelement als jeweils getrennt voneinander vorliegende Teile umfasst.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein Aspekt der Erfindung betrifft eine Vorrichtung zur Abgabe einer medizinischen Flüssigkeit an einen Patienten. Die Vorrichtung kann einen Behälter, einen Adapter und ein Abgabeelement aufweisen. Der Behälter ist bevorzugt elastisch verformbar. Der Behälter ist bevorzugt zur Aufnahme einer Flüssigkeit ausgebildet und eingerichtet. Der Behälter kann eine Flüssigkeit enthalten. Der Adapter kann mit dem Behälter fluidleitend verbindbar sein. Das Abgabeelement kann zur Abgabe einer Flüssigkeit aus der Vorrichtung ausgebildet und eingerichtet sein. Der Behälter kann ein erstes Verbindungselement aufweisen. Der Adapter kann ein zweites Verbindungselement aufweisen. Der Adapter kann ein drittes Verbindungselement aufweisen. Der Adapter kann eine erste Durchführung zur Abgabe einer Flüssigkeit aus dem Behälter aufweisen. Das Abgabeelement kann ein viertes Verbindungselement aufweisen. Das Abgabeelement kann eine zweite Durchführung zur Abgabe einer Flüssigkeit aus der Vorrichtung aufweisen.

In einer Ausführungsform ist die Vorrichtung ausgebildet und eingerichtet, durch Eingreifen des ersten Verbindungselements in das zweite Verbindungselement und durch Eingreifen des dritten Verbindungselements in das vierte Verbindungselement eine druckfeste fluidleitende Verbindung zwischen dem Behälter, dem Adapter und dem Abgabeelement herzustellen.

In einer Ausführungsform ist die Vorrichtung ausgebildet und eingerichtet, eine Flüssigkeit durch Zusammendrücken des Behälters mit einem Druck zu beaufschlagen, und hierdurch die Flüssigkeit über die erste Durchführung des Adapters und die zweite Durchführung des Abgabeelements aus der Vorrichtung an einen Patienten abzugeben.

In einer Ausführungsform weist die Vorrichtung zur Abgabe einer medizinischen Flüssigkeit an einen Patienten einen elastisch verformbaren Behälter zur Aufnahme einer Flüssigkeit,
einen Adapter, der mit dem Behälter fluidleitend verbindbar ist,
und ein Abgabeelement zur Abgabe einer Flüssigkeit aus der Vorrichtung auf,
wobei der Behälter ein erstes Verbindungselement aufweist,
wobei der Adapter ein zweites Verbindungselement, ein drittes Verbindungselement, und eine erste Durchführung zur Abgabe einer Flüssigkeit aus dem Behälter aufweist,
wobei das Abgabeelement ein viertes Verbindungselement und eine zweite Durchführung zur Abgabe einer Flüssigkeit aus der Vorrichtung aufweist,
wobei die Vorrichtung ausgebildet und eingerichtet ist, durch Eingreifen des ersten Verbindungselements in das zweite Verbindungselement und durch Eingreifen des dritten Verbindungselements in das vierte Verbindungselement eine druckfeste fluidleitende Verbindung zwischen dem Behälter, dem Adapter und dem Abgabeelement herzustellen,
und wobei die Vorrichtung ausgebildet und eingerichtet ist, eine Flüssigkeit durch Zusammendrücken des Behälters mit einem Druck zu beaufschlagen, und hierdurch die Flüssigkeit über die erste Durchführung des Adapters und die zweite Durchführung des Abgabeelements aus der Vorrichtung an einen Patienten abzugeben.

Eine Ausführungsform betrifft eine Vorrichtung zur Abgabe einer medizinischen Flüssigkeit an einen Patienten, aufweisend
einen elastisch verformbaren Behälter zur Aufnahme einer Flüssigkeit,
einen Adapter, der mit dem Behälter fluidleitend verbindbar ist,
ein Abgabeelement zur Abgabe einer Flüssigkeit aus der Vorrichtung,
und ein Absaugelement zur Abführung einer Flüssigkeit,
wobei die Vorrichtung ausgebildet und eingerichtet ist, eine Flüssigkeit durch Zusammendrücken des Behälters mit einem Druck zu beaufschlagen, und hierdurch die Flüssigkeit aus der Vorrichtung an einen Patienten abzugeben, und gleichzeitig durch das Absaugelement die abgegebene Flüssigkeit von dem Patienten abzuführen.

Die erfindungsgemäße Vorrichtung ist zur Abgabe einer medizinischen Flüssigkeit an einen Patienten geeignet. Die Vorrichtung ist insbesondere zur Reinigung bakteriell infizierten Gewebes vorgesehen, beispielsweise während eines chirurgischen Eingriffs. Die Vorrichtung kann bevorzugt zur Reinigung infizierter Implantate verwendet werden.

Die Vorrichtung ist bevorzugt geeignet, Anhaftungen an äußeren und inneren Wunden, wie z.B. Blut, Wundsekret und Debris (d.h. Gewebetrümmer), wirksam zu entfernen.

Die Vorrichtung ist bevorzugt geeignet, eine Flüssigkeit an ein Gewebe eines Patienten abzugeben, und die zur Reinigung verwendete Flüssigkeit abzuführen, um eine Kontamination eines benachbarten Patientengewebes zu vermeiden. Hierbei kann es sich entweder um ein oberflächliches oder ein inneres Patientengewebe handeln. Beispielsweise kann die Vorrichtung zur Behandlung von Geschwüren und ähnlichen äußeren Wunden im Bereich der Epidermis oder Dermis geeignet sein. Die Vorrichtung kann zur Behandlung eines inneren Patientengewebes, wie z.B. chirurgischen Wunden, geeignet sein, insbesondere im Bereich eines Gelenks oder Gelenkimplantats.

Eine "Flüssigkeit", insbesondere eine "medizinische Flüssigkeit", wie hierin beschrieben, ist bevorzugt eine biokompatible Flüssigkeit, d. h., sie ist für den unmittelbaren Kontakt mit Körpergewebe verträglich. Insbesondere ist eine hierin beschriebene medizinische Flüssigkeit geeignet zur Reinigung einer chirurgischen Wunde, beispielsweise eines infizierten Gelenks oder Gelenkimplantats. Die Flüssigkeit kann eine wässrige Lösung mit molekular dispergierten, d. h. gelösten, Bestandteilen sein. Die Flüssigkeit ist bevorzugt sichtklar. Die Vorrichtung kann auch mit wässrigen Dispersionen und Emulsionen verwendet werden. Die Flüssigkeit ist bevorzugt steril, was hierin nachfolgend weiter ausgeführt ist.

Ein "Patient" kann ein Mensch oder ein Tierpatient sein. Demzufolge kann die erfindungsgemäße Vorrichtung in der Humanmedizin und/oder in der Tiermedizin Verwendung finden.

Die erfindungsgemäße Vorrichtung weist einen elastisch verformbaren Behälter auf, der zur Aufnahme einer Flüssigkeit, insbesondere einer medizinischen Flüssigkeit, geeignet ist. Beispiele für geeignete Behälter umfassen Kunststoffflaschen und Folienbeutel. Ein "elastisch verformbarer Behälter" bedeutet im Zusammenhang mit der vorliegenden Erfindung, dass der Behälter mit manueller Kraft zusammengedrückt werden kann, um eine darin enthaltene Flüssigkeit aus dem Behälter abzugeben. Derartige Behälter finden beispielsweise als Spritzflaschen in Laboratorien oder medizinischen Behandlungseinrichtungen Verwendung. Der Behälter kann einen Kunststoff aufweisen, oder aus Kunststoff bestehen. Der Kunststoff kann ein thermoplastischer Kunststoff sein. Beispiele für verwendbare Kunststoffe umfassen Polyethylen, Polypropylen und Polyethylenterephthalat.

Der Behälter kann einen kreisförmigen oder elliptischen Querschnitt aufweisen. Der Behälter kann einen Außendurchmesser im Bereich von 7 cm bis 13 cm aufweisen. Eine solche Größe erlaubt eine gute manuelle Handhabung. Der Behälter kann eine Wandstärke von 1,5 mm bis 4,0 mm aufweisen. Dies erlaubt eine manuelle Verformung des Behälters, sodass dieser von einem Benutzer zusammengedrückt werden kann, um eine darin enthaltene Flüssigkeit ohne weitere Hilfsmittel mit Druck zu beaufschlagen. Weiterhin lässt sich durch eine solche Wandstärke insbesondere mit den oben genannten und ähnlichen Materialien eine elastische Rückstellkraft erzeugen, sodass der Behälter nach einem Zusammendrücken durch den Benutzer selbsttätig seine ursprüngliche Form wieder annehmen kann.

In einer Ausführungsform ist der Behälter versiegelt, insbesondere keimdicht versiegelt. Hierzu kann der Behälter, wie an anderer Stelle ausführlicher dargelegt, ein Septum aufweisen. Alternativ oder zusätzlich kann der Behälter mit einer Folie versiegelt sein. In einer Ausführungsform weist der Behälter ein Septum auf, welches durch eine unmittelbar darauf angebrachte Folie versiegelt ist.

Der Behälter kann einen Schraubverschluss aufweisen, der zur flüssigkeitsdichten Versiegelung des Behälters ausgebildet und eingerichtet ist. Der Schraubverschluss ist bevorzugt lösbar mit dem Behälter verbindbar. Der Schraubverschluss kann ein separates Deckelteil aufweisen, oder mit einem solchen Deckelteil verbindbar sein.

Die erfindungsgemäße Vorrichtung weist weiterhin einen Adapter auf, der mit dem Behälter fluidleitend verbindbar ist. Der Adapter kann beispielsweise die Form einer Kappe aufweisen, und zur Anbringung an eine Öffnung des Behälters eingerichtet sein. Beispielsweise kann der Adapter auf den Hals oder auf ein Kopfteil einer Kunststoffflasche anbringbar sein. Der Adapter dient erfindungsgemäß zur Herstellung einer druckfesten fluidleitenden Verbindung zwischen dem Behälter und einem hierin beschriebenen Abgabeelement. Eine "druckfeste fluidleitende Verbindung" beschreibt erfindungsgemäß, dass die betreffenden Elemente der Vorrichtung, insbesondere der Behälter, der Adapter und das Abgabeelement, fluidleitend miteinander in Verbindung bleiben, wenn eine Flüssigkeit durch Zusammendrücken des Behälters mit einem Druck beaufschlagt wird, beispielsweise mit einem Druck von 1 bar oberhalb des atmosphärischen Drucks außerhalb der Vorrichtung, um die Flüssigkeit aus der Vorrichtung abzugeben. Der Begriff "druckfeste fluidleitende Verbindung" impliziert weiterhin, dass die Flüssigkeit nicht in unerwünschter Weise zwischen dem Behälter und dem Adapter oder zwischen dem Adapter und dem Abgabeelement aus der Vorrichtung austreten kann. In anderen Worten kann eine druckfeste fluidleitende Verbindung unerwünschte Leckagen der Vorrichtung verhindern, und sicherstellen, dass die aus dem Behälter abzugebende Flüssigkeit ausschließlich über das Abgabeelement freigesetzt werden kann.

Zur Herstellung einer solchen druckfesten fluidleitenden Verbindung kann der Behälter erfindungsgemäß ein erstes Verbindungselement aufweisen, welches zum Eingriff in ein zweites Verbindungselement angeordnet und eingerichtet ist, wobei das zweite Verbindungselement an dem Adapter angeordnet ist. In ähnlicher Weise kann an dem Adapter weiterhin ein drittes Verbindungselement angeordnet sein, welches zum Eingriff in ein viertes Verbindungselement angeordnet und eingerichtet ist, wobei das vierte Verbindungselement an dem Abgabeelement angeordnet ist.

In einer Ausführungsform weist das erste Verbindungselement ein Rastelement auf. In einer Ausführungsform weist das zweite Verbindungselement ein Rastelement auf. In einer Ausführungsform weisen das erste Verbindungselement und das zweite Verbindungselement jeweils ein Rastelement auf. Wie hierin verwendet, bezieht sich der Begriff "Rastelement" auf eine Struktur, die ausgebildet und eingerichtet ist, an einer vordefinierten Stelle mit einem anderen Element in Eingriff zu treten, und dadurch eine mechanische Verbindung herzustellen, welche gegen ein versehentliches Lösen gesichert ist. Bevorzugt sind das erste und das zweite Verbindungselement ausgebildet und eingerichtet, ineinander einzugreifen, um eine formschlüssige und/oder kraftschlüssige Verbindung herzustellen. In einer Ausführungsform ist das Rastelement elastisch verformbar, insbesondere biegsam.

In einer Ausführungsform ist das erste Verbindungselement als biegsamer Haken ausgestaltet, und das zweite Verbindungselement ist als Vorsprung ausgestaltet, in welchen der Haken eingreifen kann. In einer Ausführungsform ist das zweite Verbindungselement als biegsamer Haken ausgestaltet, und das erste Verbindungselement ist als Vorsprung ausgestaltet, in welchen der Haken eingreifen kann. In einer Ausführungsform weist der Adapter mehrere Haken zur Verbindung mit dem Behälter auf, und der Behälter weist ein oder mehrere Vorsprünge auf, in welche die Haken eingreifen können. Der Vorsprung kann beispielsweise als umlaufender Ring an dem Behälter oder dem Adapter ausgebildet sein.

In einer Ausführungsform sind das erste und das zweite Verbindungselement jeweils als Gewinde ausgebildet.

In einer Ausführungsform weist die Vorrichtung einen Bajonettverschluss auf, der zur Verbindung des Adapters mit dem Behälter ausgebildet und eingerichtet ist.

In einer Ausführungsform weist die Vorrichtung einen Bajonettverschluss auf, der zur Verbindung des Adapters mit dem Abgabeelement ausgebildet und eingerichtet ist.

In einer Ausführungsform ist das Rastelement ausgebildet und eingerichtet, den Behälter und den Adapter druckfest fluidleitend miteinander zu verbinden, wenn der Adapter mit dem Behälter in Kontakt gebracht wird und der Adapter gegen den Behälter gedrückt wird. Beispielsweise kann das erste Verbindungselement als Haken ausgestaltet sein, welcher ausgebildet und eingerichtet ist, in ein zweites Verbindungselement, das als Vorsprung ausgebildet ist, einzugreifen, wenn der Adapter in Kontakt mit dem Behälter gebracht und gegen den Behälter gedrückt wird. Somit kann der Adapter mit dem Behälter verbunden und verrastet werden, sodass diese beiden Teile eine druckfeste fluidleitende Verbindung miteinander ausbilden. Somit kann eine leckfreie, d. h. in Bezug auf die Verbindung des Behälters mit dem Adapter flüssigkeitsdichte, Abgabe einer Flüssigkeit aus dem Behälter in die erste Durchführung des Adapters sichergestellt werden.

Die Vorrichtung kann weiterhin ein gummielastisches Dichtelement aufweisen. Das Dichtelement ist bevorzugt an dem Adapter oder an dem Abgabeelement angeordnet. Das Dichtelement kann die Ausbildung einer druckfesten und fluidleitenden Verbindung zwischen Adapter und Abgabeelement weiter verbessern. Das Dichtelement ist demnach in einer Ausführungsform ausgebildet und eingerichtet, eine flüssigkeitsdichte Verbindung zwischen dem Adapter und dem Abgabeelement auszubilden.

Beispielsweise kann der Adapter eine ringförmige Dichtung aufweisen. Die ringförmige Dichtung kann nach Art eines "O-Rings" ausgebildet sein. Das Dichtelement kann in einer umlaufenden Rille des Adapters angeordnet sein. Das Dichtelement kann auch eine scheibenförmige Form aufweisen. Das Dichtelement kann daher nach Art einer flächigen Dichtung, die auch als "Flachdichtung" bezeichnet wird, ausgebildet sein. Ein solches Dichtelement kann auf einer Außenfläche des Adapters oder des Abgabeelements aufliegend angeordnet sein. In einer Ausführungsform weist die Vorrichtung ein Dichtelement auf, um das erste Verbindungselement, das zweite Verbindungselement, das dritte Verbindungselement, das vierte Verbindungselement und/oder das fünfte Verbindungselement bei der Ausbildung einer flüssigkeitsdichten Verbindung zu unterstützen. Das Dichtelement kann dabei an jeder geeigneten Stelle der Vorrichtung angebracht sein, insbesondere am Adapter oder an dem Abgabeelement. Das "gummielastisches Dichtelement" bezeichnet hierin insbesondere ein Element der Vorrichtung, welches sich von dem hierin beschriebenen "Septum" in Bezug auf seine Form und/oder Anordnung an der Vorrichtung unterscheidet.

Der Adapter kann eine erste Durchführung aufweisen, um eine Flüssigkeit aus dem Behälter an das Abgabeelement abzugeben. Das Abgabeelement kann eine zweite Durchführung aufweisen, um eine Flüssigkeit aus dem Adapter aufzunehmen, und aus der Vorrichtung abzugeben. Die erste Durchführung erstreckt sich bevorzugt durch einen Innenraum des Adapters. Die zweite Durchführung erstreckt sich bevorzugt durch einen Innenraum des Abgabeelements. Die erste Durchführung und/oder die zweite Durchführung können insbesondere als innenliegende Hohlräume oder Kanäle ausgebildet sein. Die erste Durchführung und/oder die zweite Durchführung können unmittelbar durch das Material des Adapters bzw. des Abgabeelements gebildet sein, oder können jeweils eine eigene Schlauchleitung oder Rohrleitung aufweisen.

Die erste Durchführung kann einen Innendurchmesser (d. h. lichte Weite) von 0,5 mm bis 3 mm aufweisen. Die zweite Durchführung kann einen Innendurchmesser von 0,5 bis 3 mm aufweisen.

In einer Ausführungsform weist die Vorrichtung weiterhin ein Septum auf, welches den Behälter flüssigkeitsdicht verschließt. Das Septum kann bevorzugt an dem Behälter angeordnet sein. Beispielsweise kann das Septum an einer Öffnung des Behälters angeordnet sein. Das Septum ist bevorzugt flüssigkeitsdicht mit dem Behälter verbunden. Das Septum kann kraftschlüssig, z.B. mithilfe einer Halterung, mit dem Behälter verbunden sein. Das Septum kann stoffschlüssig mit dem Behälter verbunden sein, z.B. durch eine Schmelzverbindung (z.B. Folienschweißung). In einigen Ausführungsformen weist das Septum ein flexibles, selbstdichtendes Polymer auf. Das Polymer kann ein polyhalogeniertes Olefin, ein Silikon, oder ein thermoplastisches Elastomer aufweisen. In einer Ausführungsform umfasst das Septum Butylkautschuk. In einer Ausführungsform umfasst das Septum Silikonkautschuk. Das Septum ist bevorzugt eingerichtet, nach einem Durchstechen des Septums mit einer Kanüle das Gehäuse flüssigkeitsdicht zu verschließen, mit Ausnahme der Auslässe des Gehäuses. Das Septum ist bevorzugt dehnbar und gummielastisch, wie es beispielsweise von Septen in Chemikalienflaschen bekannt ist. Dadurch kann sich das Septum bevorzugt nach einem Durchstich selbst heilen.

Bevorzugt weist das Septum ein biokompatibles Material auf.

Das Septum kann beispielsweise eine im Wesentlichen flache oder eine konvex gewölbte Form aufweisen. Das Septum kann eine kreisförmige Form aufweisen.

In einer Ausführungsform weist das Septum ein Material auf, das mit einer medizinischen Injektionskanüle gemäß der Norm ISO 7864:2016 mit einer Kraft von weniger als 100 N durchstechbar ist.

Das Septum kann mit einer Abdeckung versehen sein, welche ein oder mehrere Öffnungen aufweist. Durch eine solche Öffnung kann ein Benutzer der Vorrichtung den erfindungsgemäßen Adapter fluidleitend mit dem Behälter verbinden. Die Abdeckung kann in die oben genannte Halterung des Septums integriert sein.

Weiterhin können entsprechende Öffnungen an geeigneten Stellen des Adapters vorgesehen sein, die eine erste Durchstichposition und gegebenenfalls eine zweite Durchstichposition definieren. Die erste Durchstichposition kann zur Einführung eines Hohldorns durch das Septum in den Behälter ausgebildet sein, wobei der Hohldorn bevorzugt an dem Adapter angeordnet ist.

Falls die Abdeckung mehrere Öffnungen aufweist, können zusätzlich weitere Zubehörteile fluidleitend mit dem Behälter verbunden werden. Zu diesem Zweck kann ein spitzer Anschluss, wie z.B. ein Hohldorn, durch eine zweite Öffnung der Abdeckung durch das Septum geführt werden. Die oben genannte zweite Durchstichposition kann somit zum Anschluss eines Zubehörteils ausgebildet sein.

An dem Septum kann weiterhin eine Kunststoffmembran angeordnet sein, die geeignet ist, einen direkten Kontakt einer in dem Behälter enthaltenen Flüssigkeit mit dem Septum zu verhindern, solange das Septum unverletzt ist.

Zubehörteile, die in Verbindung mit der vorliegenden Erfindung verwendbar sind, umfassen beispielsweise einen Schlauch, einen Spritzschutzschirm oder eine Lavagevorrichtung. Die hierin beschriebenen Vorrichtungen sind in einigen Ausführungsformen zum Betrieb mit einer externen Lavagevorrichtung geeignet. In einer Ausführungsform kann eine solche externe Lavagevorrichtung wie oben geschildert über eine zweite Öffnung an einer zweiten Durchstichposition des Septums mit der Vorrichtung verbunden werden. Beispiele für Lavagevorrichtungen, mit denen die hierin beschriebenen Vorrichtungen betrieben werden können, sind beispielsweise in EP2662146A2, US4,583,531A, US4,278,078A und US5,542,918A beschrieben. Solche Lavagevorrichtungen können eine Pumpe umfassen, die beispielsweise mit einem Elektromotor oder einem Druckluftmotor ausgestattet sein kann. Bevorzugt können die hierin beschriebenen Vorrichtungen mit Lavagevorrichtungen betrieben werden, welche gepulste Sprühstöße erzeugen können. Die Lavagevorrichtungen können beispielsweise Pumpen umfassen, wie sie in EP2619415A1, EP2910270A1 oder EP2873856A1 beschrieben sind.

Zum Anschluss an eine solche Lavagevorrichtung kann die hierin beschriebene Vorrichtung mit einem geeigneten weiteren Verbindungselement ausgestattet sein. Ein solches Verbindungselement kann formschlüssig oder kraftschlüssig mit einer externen Lavagevorrichtung verbindbar sein. Beispielsweise kann das Verbindungselement ein Rastelement aufweisen, um die Vorrichtung mit einer Lavagevorrichtung zu verbinden, bevorzugt druckfest fluidleitend zu verbinden.

In einer Ausführungsform weist der Adapter weiterhin eine stielförmige Düse auf. Bevorzugt ist die stielförmige Düse fluidleitend mit der ersten Durchführung verbunden. In einer Ausführungsform ist die Düse ausgebildet und angeordnet, sich nach außen zu erstrecken, wenn der Adapter durch Eingreifen des ersten Verbindungselements in das zweite Verbindungselement mit dem Behälter verbunden ist. Mit anderen Worten kann die Düse sich als längliches Element von dem Adapter in Richtung des Abgabeelements erstrecken, bzw. in Richtung des Teils des Adapters, das zur Verbindung mit dem Abgabeelement vorgesehen ist. Die stielförmige Düse des Adapters kann somit zur Abgabe einer Flüssigkeit aus dem Behälter dienen. Weiterhin kann die stielförmige Düse des Adapters bevorzugt formschlüssig in eine Düse eines Abgabeelements der Vorrichtung einführbar sein.

Die stielförmige Düse kann die Form eines Zylinders aufweisen, bevorzugt die Form eines abgerundeten Zylinders. Die stielförmige Düse ist in einer Ausführungsform zur Abgabe einer Flüssigkeit aus dem Behälter ausgebildet und eingerichtet. Die Düse kann eingerichtet sein, eine Flüssigkeit an das Abgabeelement abzugeben. Die Düse kann eingerichtet sein, eine Flüssigkeit an einen Patienten abzugeben. Die Düse kann einen im Wesentlichen zylindrischen, an einem Ende spitz zulaufenden Hohlkörper aufweisen. Die Düse kann einen zylinderförmigen Grundkörper aufweisen, welcher an einem Ende des Grundkörpers in eine spitz zulaufende, abgerundete Spitze übergeht.

In einer Ausführungsform ist die stielförmige Düse des Adapters ausgebildet und eingerichtet, mit der zweiten Durchführung eine druckfeste fluidleitende Verbindung herzustellen, wenn der Adapter durch Eingreifen des dritten Verbindungselements in das vierte Verbindungselement mit dem Abgabeelement verbunden ist. Hierdurch kann erreicht werden, dass keine Flüssigkeit unerwünscht zwischen dem Adapter und dem Abgabeelement aus der Vorrichtung austritt, wenn der Behälter mit Druck beaufschlagt wird, um eine Flüssigkeit aus dem Behälter aus der Vorrichtung freizusetzen. Weiterhin kann hierdurch sichergestellt werden, dass sich hierbei das Abgabeelement nicht unerwünscht von der Vorrichtung löst, sondern eine feste Verbindung zwischen dem Adapter und dem Abgabeelement beibehalten wird.

Der Adapter kann eine Kappe mit der Form eines C-förmigen Querschnitts aufweisen. Der Adapter kann ausgebildet und eingerichtet sein, den Behälter von mehreren Seiten formschlüssig zu umgeben. Der Adapter kann zur Herstellung eines Formschlusses mit dem Behälter ausgebildet und eingerichtet sein, wobei dieser Formschluss bevorzugt unabhängig von dem ersten Verbindungselement und/oder dem zweiten Verbindungselement herstellbar ist. Demnach kann der Adapter beispielsweise in einer Ausführungsform durch einen ersten Formschluss mit dem Behälter verbindbar sein, der durch die Form eines C-förmigen Querschnitts des Adapters herstellbar ist, und weiterhin durch einen zweiten Formschluss mit dem Behälter verbindbar sein, der durch das erste Verbindungselement und das zweite Verbindungselement herstellbar ist.

Der Adapter kann einen Hohldorn aufweisen. Dies ermöglicht das Durchstechen eines Septums zur Herstellung einer fluidleitenden Verbindung zwischen Behälter und Adapter. Der Hohldorn ist bevorzugt fluidleitend mit der ersten Durchführung verbunden. Der Hohldorn weist bevorzugt einen Innenraum auf, der das Hindurchleiten einer Flüssigkeit durch den Hohldorn erlaubt.

Der Innenraum kann beispielsweise einen Durchmesser von 1 mm bis 3 mm aufweisen. Der Hohldorn kann eine Auslassöffnung mit einen Durchmesser kleiner 1,5 mm und besonders bevorzugt kleiner 1 mm aufweisen. Der Hohldorn kann eine angeschrägte Spitze, ähnlich einer geschliffenen Kanüle, aufweisen, wobei sich bevorzugt in einer Schräge der Spitze eine Auslassöffnung des Hohldorns befindet. Der Hohldorn kann eine abgerundete Spitze aufweisen. Hierbei können nahe der der Spitze ein oder mehrere Öffnungen angeordnet sein, die mit dem Innenraum des Hohldorns verbunden sind. Der Hohldorn kann aus Kunststoff oder Metall, z.B. Edelstahl, ausgebildet sein. Beispiele für verwendbare Kunststoffe umfassen Polyethylen, Polypropylen und Polyethylenterephthalat, Polyamid 12, Polyamid 6, Polyamid 66 und Polyethersulfon. Der Hohldorn, insbesondere die Spitze des Hohldorns, kann durch eine abnehmbare Schutzkappe bedeckt sein.

Der Hohldorn kann eine Länge von 0,8 cm bis 3,0 cm aufweisen. Ein solcher Hohldorn ist vorteilhaft, wenn die Vorrichtung bei der Benutzung "über Kopf", d. h. in einer Anordnung, in der der Behälter nach oben (entgegen der Schwerkraftrichtung) ausgerichtet ist, gebraucht wird. Der Hohldorn kann eine Länge aufweisen, welche eine Anordnung der Spitze des Hohldorns in der Nähe des Bodens des Behälters ermöglicht. Der Hohldorn kann auch eine Länge aufweisen, welche ca. 70 % bis 90 % der Länge des Behälters entspricht. Ein solcher Hohldorn ist vorteilhaft, wenn die Vorrichtung "aufrecht", d. h. in einer Anordnung in der der Behälter nach unten (in Schwerkraftrichtung) ausgerichtet ist, gebraucht wird. Dies kann hilfreich sein, um eine in dem Behälter enthaltene Flüssigkeit möglichst vollständig über den Hohldorn nach außen zu führen.

In einer Ausführungsform ist das Abgabeelement ausgebildet und eingerichtet, den Adapter nach außen vollständig zu bedecken, um den Adapter vor einer äußeren Kontamination zu schützen. Dies kann bewirken, dass der Adapter bei bestimmungsgemäßem Verbrauch der Vorrichtung steril, oder zumindest besser vor Kontamination geschützt, bleibt, wenn das Abgabeelement an dem Adapter angeordnet ist. Dies ermöglicht dem Benutzer der Vorrichtung, ein kontaminiertes Abgabeelement gegen ein neues, sauberes und bevorzugt steriles neues Abgabeelement auszuwechseln, und dabei die übrigen Teile der Vorrichtung weiter zu verwenden, insbesondere während der Behandlung desselben Patienten, z.B. wenn verschiedene Positionen des Patienten mit der erfindungsgemäßen Vorrichtung zu behandeln sind.

Das Abgabeelement kann eine Form aufweisen, die zu einer Oberfläche des Adapters komplementär ist. Dies kann eine formschlüssige Verbindung des Abgabeelements mit dem Adapter ermöglichen. Das Abgabeelement kann eine Kappe mit der Form eines C-förmigen Querschnitts aufweisen. Das Abgabeelement kann ausgebildet und eingerichtet sein, den Adapter von mehreren Seiten formschlüssig zu umgeben. Das Abgabeelement kann zur Herstellung eines Formschlusses mit dem Adapter ausgebildet und eingerichtet sein, wobei dieser Formschluss unabhängig von dem dritten Verbindungselement und/oder dem vierten Verbindungselement herstellbar ist.

In einer Ausführungsform weist das Abgabeelement weiterhin ein fünftes Verbindungselement auf, welches unabhängig von dem dritten Verbindungselement und dem vierten Verbindungselement mit einem Zubehörteil verbindbar ist. In einer Ausführungsform ist das Zubehörteil ausgewählt aus der Gruppe bestehend aus einem Schlauch, einem Spritzschutzschirm, einer Lavage-Vorrichtung und einer Reinigungsbürste. Das fünfte Verbindungselement kann beispielsweise einen Schlauchanschluss aufweisen. Ein Schlauch kann mit dem Abgabeelement verbindbar sein, beispielsweise um mithilfe der Vorrichtung abgegebene und verunreinigte Flüssigkeit und/oder zu entfernendes Gewebe von dem Patienten abzuführen. Bei dem zu entfernenden Gewebe kann sich beispielsweise um mikrobiell infiziertes Gewebe eines Patienten handeln, das bei einer Behandlung eines infizierten Gelenks oder Implantats, insbesondere einer DAIR-Behandlung, entfernt wird.

Ein Verbindungselement, das zur Verbindung mit einem Schlauch vorgesehen ist, insbesondere das fünfte Verbindungselement der Vorrichtung, kann bevorzugt ein oder mehrere Vorsprünge oder Widerhaken aufweisen, um ein versehentliches Lösen des Schlauchs von dem Verbindungselement zu verhindern. Ein solches Verbindungselement kann fest mit der Vorrichtung verbunden sein, oder kann lösbar mit der Vorrichtung verbindbar sein. Beispielsweise kann die Vorrichtung einen Schlauchverbinder zum Anschluss eines Schlauchs der Vorrichtung mit einem weiteren Schlauch aufweisen. Solche Schlauchverbinder werden auch als "Schlauchkupplung" bezeichnet. Sie können jeweils an zwei gegenüberliegenden Seiten stielförmige Anschlüsse für einen Schlauch aufweisen, wobei die Anschlüsse jeweils Vorsprünge oder Widerhaken aufweisen können, um einen Schlauch kraftschlüssig in Position zu halten.

Ein Spritzschutzschirm kann eine Vorrichtung aufweisen, die eine unerwünschte Verteilung von mikrobiell belasteter Flüssigkeit verringern oder verhindern kann. Der Spritzschutzschirm kann die Form einer Scheibe aufweisen. Der Spritzschutzschirm kann eine konkave Krümmung aufweisen, ähnlich einer Parabolantenne. Der Spritzschutzschirm kann eine kegelförmige Form aufweisen. Der Spritzschutzschirm kann eine zentral angeordnete Ausnehmung aufweisen, die zur Anbringung des Spritzschutzschirms an einem Abgabeelement der Vorrichtung ausgebildet und eingerichtet ist. Der Spritzschutzschirm kann einen Kunststoff aufweisen. Der Spritzschutzschirm kann transparent oder transluzent sein. Der Kunststoff kann ein Elastomer aufweisen.

Geeignete Reinigungsbürsten, die mit der erfindungsgemäßen Vorrichtung verbindbar sind, sind hierin an anderer Stelle ausführlicher beschrieben. Die Reinigungsbürsten können eines oder mehrere der Merkmale aufweisen, die hierin im Zusammenhang mit einer "Reinigungsbürste" oder einem "Bürstenkopf" beschrieben sind. Die Vorrichtung kann eine fest mit einem Abgabeelement der Vorrichtung verbundene Reinigungsbürste, wie z.B. einen hierin beschriebenen Bürstenkopf, oder eine lösbar mit der Vorrichtung verbindbare Reinigungsbürste aufweisen.

In einer Ausführungsform ist der Behälter mit einer sterilen Flüssigkeit befüllt. Die Flüssigkeit ist bevorzugt eine wässrige Flüssigkeit. Bevorzugt umfasst die Flüssigkeit einen antiseptischen Wirkstoff. Geeignete antiseptische Wirkstoffe umfassen kationische, anionische, neutrale und oxidierende Wirkstoffe.

Beispiele für kationische Wirkstoffe sind Benzalkoniumchlorid (CAS 8001-54-5), Octenidindihydrochlorid (CAS 70775-75-6), Polyhexanid (CAS 32289-58-0) und Chlorhexidindiglukonat (CAS 18472-5-0). Ein beispielhafte Vertreter der anionischen Antiseptika ist Natriumdodecylsulfat (CAS 151-21-3). Beispielhafte oxidierende Wirkstoffe sind Natriumhypochlorit (CAS 10022-70-5), zum Beispiel in Form der Dakin'schen Lösung, Natriumchlorit (CAS 49658-21-1) -haltige wässrige Lösungen und Jod (CAS 7553-56-2), beispielsweise in Form von Jod-Kaliumjod (CAS 7681-11-0) oder Povidon-Jod (CAS 25655-41-8).

In einer Ausführungsform ist der antiseptische Wirkstoff ausgewählt aus der Gruppe bestehend aus Chlorhexidinglukonat, Benzalkoniumchlorid, Octenidindihydrochlorid, einem Alkalihypochlorit, einem Erdalkalihypochlorit, und einem Alkalichlorit. In einer Ausführungsform umfasst die Flüssigkeit Natriumdodecylsulfat oder eine physiologische Kochsalzlösung.

In einer Ausführungsform umfasst die Flüssigkeit eine wässrige Lösung, die ausgewählt ist aus der Gruppe bestehend aus
einer Lösung von Benzalkoniumchlorid mit einer Benzalkoniumchlorid-Konzentration von 50 bis 1500 ppm;
einer Lösung von Chlorhexidindiglukonat und Benzalkoniumchlorid, wobei die Konzentration an Chlorhexidindiglukonat 50 bis 600 ppm beträgt und die Konzentration an Benzalkoniumchlorid 50 bis 250 ppm beträgt;
einer Lösung von Octenidindihydrochlorid mit einer Konzentration von 50 bis 2000 ppm;
einer Lösung eines Alkalihypochlorits mit einer Konzentration an Hypochlorit von 50 bis 2000 ppm;
einer Lösung eines Erdalkalihypochlorits mit einer Konzentration an Hypochlorit 50 bis 2000 ppm;
einer Lösung eines Alkalichlorits mit einer Konzentration an Chlorit von 50 bis 2000 ppm,
einer Lösung von Natriumdodecylsulfat mit einer Konzentration an Natriumdodecylsulfat von 50 bis 2000 ppm; und
einer physiologischen Kochsalzlösung mit einer Konzentration an Natriumchlorid von 0,9 Gew.-%.

Soweit nicht ausdrücklich anders angegeben, beziehen sich die obigen und sonstigen hierin genannten Konzentrationsangaben in % oder ppm jeweils auf einen Masse/Masse-Anteil im Verhältnis der jeweils genannten gelösten Stoffe zur Gesamtmasse der wässrigen Lösung.

In einer Ausführungsform weist die Flüssigkeit eine Puffersubstanz auf. In einer Ausführungsform ist die Flüssigkeit frei von Puffersubstanzen. In einer Ausführungsform ist die Puffersubstanz ausgewählt aus der Gruppe bestehend aus Citronensäure (CAS 77-92-9), Natriumdihydrogencitrat (CAS 18996-35-5), Dinatriumhydrogencitrat (CAS 6132-05-4), Natriumdihydrogenphosphat (CAS 7558-80-7), Calciumdihydrogenphosphat (CAS 7758-23-8), Natriumhydrogensulfat (CAS 7681-38-1), Dinatriumhydrogenphosphat (CAS 7558-79-4) und Trinatriumphosphat (CAS 760-54-9).

In einer Ausführungsform besteht die Flüssigkeit aus Reinstwasser und einem Wirkstoff. In einer Ausführungsform besteht die Flüssigkeit aus Reinstwasser und zwei Wirkstoffen. In einer Ausführungsform ist das Reinstwasser Wasser für Injektionszwecke.

In einer Ausführungsform ist die Flüssigkeit frei von organischen Lösungsmitteln. In einer Ausführungsform enthält die Flüssigkeit außer Wasser keinen anderen flüssigen Stoff. In diesem Zusammenhang sind gelöste Feststoffe oder Gase, insbesondere dissoziierte Salze, nicht als "flüssiger Stoff" zu verstehen.

In einer weiteren Ausführungsform wird die erfindungsgemäße Vorrichtung als Teilesatz bereitgestellt, wobei der Teilesatz einen hierin beschriebenen Behälter, einen hierin beschriebenen Adapter und ein hierin beschriebenes Abgabeelement als jeweils getrennt voneinander vorliegende Teile umfasst. In einer Ausführungsform wird der Teilesatz in einem versiegelten, sterilen Packmittel bereitgestellt. Demnach betrifft die Erfindung auch einen Teilesatz in einem versiegelten, sterilen Packmittel, welches in einem sterilen Innenraum des Packmittels einen hierin beschriebenen Behälter, einen hierin beschriebenen Adapter, ein hierin beschriebenes Abgabeelement und gegebenenfalls weitere Teile der Vorrichtung oder Zubehörteile davon aufweist.

Der Teilesatz kann ein oder mehrere Abgabeelemente aufweisen, die hierin beschrieben sind. Beispielsweise kann der Teilesatz mehrere unterschiedlich geformte Abgabeelemente aufweisen.

Der Teilesatz kann mehrere Zubehörteile aufweisen.

In einigen Ausführungsformen ist die Vorrichtung sterilisierbar. Dies bedeutet, dass die Vorrichtung einem Sterilisationsverfahren unterzogen werden kann, ohne deren Materialeigenschaften, Verträglichkeit und Funktionsweise signifikant zu beeinträchtigen. Solche Sterilisationsverfahren umfassen beispielsweise Autoklavierung, Bestrahlung mit Elektronenstrahlen oder Gammastrahlen, oder Behandlung mit desinfizierenden Substanzen wie zum Beispiel Ethylenoxid.

In einigen Ausführungsformen ist die Vorrichtung gemäß der Norm DIN EN ISO 11135 und/oder der Norm DIN EN ISO 11137 sterilisierbar. Dies bezieht sich gegebenenfalls auch auf entsprechende Packmittel, wenn die Vorrichtung wie hierin beschrieben in einem sterilen Packmittel bereitgestellt wird. Bevorzugt ist die Vorrichtung gemeinsam mit einer gegebenenfalls enthaltenen Flüssigkeit sterilisierbar.

In einer Ausführungsform ist die Vorrichtung vollständig sterilisiert. In einer Ausführungsform ist die Vorrichtung vollständig pyrogenfrei. Der Begriff "sterilisiert" bezieht sich darauf, dass die Vorrichtung einem Verfahren unterzogen worden ist, um Krankheitserreger zu entfernen oder abzutöten, sodass die Vorrichtung gemäß der Norm DIN EN 556-1 medizinisch verwendet werden kann. Verfahren zur Sterilisation umfassen beispielsweise Autoklavierung, Bestrahlung mit Elektronenstrahlen oder Gammastrahlen, oder Behandlung mit desinfizierenden Substanzen wie zum Beispiel Ethylenoxid. In ähnlicher Weise bezieht sich der Begriff "pyrogenfrei" darauf, dass die Vorrichtung einer im Fachgebiet üblichen Behandlung unterzogen worden ist, um Pyrogene zu entfernen oder zu zersetzen. Ob die Vorrichtung pyrogenfrei ist, kann mithilfe des Limulus-Amöbozytenlysat (LAL)-Tests gemäß Ph. Eur. Kapitel 2.6.14 geprüft werden. Hierbei gelten jeweils die Normen bzw. Dokumente, die zum Prioritätstag der vorliegenden Anmeldung aktuell sind.

Wenn die Vorrichtung als Teilesatz in einem sterilen Packmittel bereitgestellt wird, gelten die obigen Ausführungen zu den Begriffen "sterilisiert" und "pyrogenfrei" in entsprechender Weise für das sterile Packmittel. In Ausführungsformen, in welchen der Behälter mit einer Flüssigkeit befüllt ist, sind Autoklavierung oder Bestrahlung mit Elektronenstrahlen oder Gammastrahlen bevorzugte Verfahren zur Sterilisation, da desinfizierenden Substanzen wie z.B. Ethylenoxid von außen nicht ohne weiteres die Wand des Behälters passieren können, um dessen Inhalt zu sterilisieren. Eine Autoklavierung umfasst die Behandlung eines zu sterilisierenden Gegenstands mit Wasserdampf bei erhöhtem Druck und erhöhter Temperatur. Beispielsweise können Temperaturen von 125 °C bis 135 °C und Drücke von 200 bis 300 kPa verwendet werden. Die Dauer der Autoklavierung kann beispielsweise 15 bis 30 Minuten betragen.

Die Vorrichtung kann ausgebildet und eingerichtet sein, wahlweise mit oder ohne das Abgabeelement eine Flüssigkeit an einen Patienten abzugeben. Somit kann die Abgabe einer Flüssigkeit an einen Patienten entweder unmittelbar durch den Adapter erfolgen, oder die Abgabe einer Flüssigkeit an einen Patienten kann durch das Abgabeelement erfolgen, wie hierin beschrieben. Dies ermöglicht einem Benutzer eine größere Flexibilität im Gebrauch der erfindungsgemäßen Vorrichtung, insbesondere im Fall einer mikrobiellen Kontamination des Abgabeelements.

In einer Ausführungsform weist die Vorrichtung ein Absaugelement zur Abführung einer Flüssigkeit auf. In einer Ausführungsform ist die Vorrichtung ausgebildet und eingerichtet, durch das Absaugelement eine Flüssigkeit von dem Patienten abzuführen.

In einer Ausführungsform ist die Vorrichtung ausgebildet und eingerichtet, eine Flüssigkeit durch Zusammendrücken des Behälters mit einem Druck zu beaufschlagen, und hierdurch die Flüssigkeit aus der Vorrichtung an einen Patienten abzugeben, und gleichzeitig durch das Absaugelement die abgegebene Flüssigkeit von dem Patienten abzuführen. Hiermit ist gemeint, dass die aus dem Abgabeelement abgegebene und zum Spülen verwendete Flüssigkeit, nachdem sie in Kontakt mit einem Patientengewebe gebracht wurde, durch das Absaugelement abgeführt werden kann.

In einer Ausführungsform sind der Behälter, der Adapter und das Abgabeelement jeweils als miteinander lösbar verbindbare Teile ausgebildet. Wie hierin bereits ausführlicher beschrieben, kann die Vorrichtung als Teilesatz bereitgestellt werden, welcher den Behälter, den Adapter und das Abgabeelement als voneinander getrennte Teile umfasst.

In anderen Ausführungsformen bilden Adapter und Abgabeelement gemeinsam eine monolithische Einheit. Dies bedeutet, dass Adapter und Abgabeelement fest miteinander verbunden sind, und durch einen Benutzer nicht voneinander getrennt werden können.

Das Absaugelement kann einen Schlauch oder ein Rohr aufweisen.

Ein Schlauch kann unmittelbar oder über einen Schlauchverbinder an der Vorrichtung angebracht sein. Der Schlauchverbinder kann lösbar mit der Vorrichtung verbindbar sein. Der Schlauchverbinder kann Vorsprünge und/oder Widerhaken aufweisen, um ein Abrutschen und Lösen des Schlauchs zu vermeiden.

Das Absaugelement kann eine Absaugöffnung aufweisen, die zur Aufnahme und Abführung einer Flüssigkeit durch das Absaugelement ausgebildet und eingerichtet ist.

In einer Ausführungsform weist die Vorrichtung eine Schelle auf, welche das Absaugelement hält. Die Schelle kann bevorzugt drehbar gelagert an der Vorrichtung angeordnet sein. In einer Ausführungsform ist die Schelle drehbar um die Längsmittelachse des Adapters gelagert.

In einer Ausführungsform weist das Absaugelement einen flexiblen oder gekrümmten Leitungsbereich auf. Beispielsweise kann das Absaugelement ein Rohr mit einem gekrümmten Leitungsbereich aufweisen.

In einer Ausführungsform weist das Absaugelement ein Gelenk auf. Das Gelenk kann beispielsweise als Scharnier oder Kugelgelenk ausgebildet sein. Das Scharnier kann ausgebildet und eingerichtet sein, das Absaugelement um eine feste Achse der Vorrichtung zu drehen. Das Kugelgelenk kann ausgebildet und eingerichtet sein, das Absaugelement um einen festen Punkt der Vorrichtung zu rotieren, wobei bevorzugt eine Drehung um mehrere verschiedene Achsen ermöglicht wird.

Die Schelle oder das Gelenk können ausgebildet und eingerichtet sein, eine selbsttätige Ausrichtung des Absaugelements zu einem tiefsten Punkt auszuführen. Das Absaugelement kann beispielsweise drehbar gelagert sein, wobei die Lagerung ausreichend leichtgängig ist, dass sich das Absaugelement durch die Einwirkung der Schwerkraft selbsttätig nach unten bewegt. Dies erleichtert es einem Anwender der Vorrichtung, eine Flüssigkeit von einem tiefsten Punkt eines zu behandelnden Patientengewebes abzuführen.

In einer Ausführungsform sind das erste Verbindungselement und das zweite Verbindungselement ausgebildet und eingerichtet, eine Drehung des Adapters relativ zu dem Behälter zu erlauben, wenn durch Eingreifen des ersten Verbindungselements in das zweite Verbindungselement eine druckfeste fluidleitende Verbindung zwischen dem Behälter und dem Adapter gebildet wird.

In einer Ausführungsform sind das dritte Verbindungselement und das vierte Verbindungselement ausgebildet und eingerichtet, eine Drehung des Abgabeelements relativ zu dem Adapter zu erlauben, wenn durch Eingreifen des dritten Verbindungselements in das vierte Verbindungselement eine druckfeste fluidleitende Verbindung zwischen dem Adapter und dem Abgabeelement gebildet wird. Dies kann beispielsweise erreicht werden, wenn das erste Verbindungselement einen umlaufenden Vorsprung aufweist, und das zweite Verbindungselement einen Rasthaken aufweist, wobei der Rasthaken entlang des Vorsprungs beweglich ist, während der Rasthaken in den Vorsprung eingreift.

Die oben geschilderten Ausführungsformen können hilfreich sein, um ein Abgabeelement und ein Absaugelement der Vorrichtung relativ zueinander in unterschiedlicher Art und Weise zu positionieren, um eine mithilfe der Vorrichtung abgegebene Flüssigkeit an der bestmöglich geeigneten Stelle nach Kontakt mit einem Patientengewebe wieder abzuführen.

In einer Ausführungsform ist die Vorrichtung ausgebildet und eingerichtet, eine Flüssigkeit an einer ersten Position abzugeben, und die abgegebene Flüssigkeit an einer zweiten Position aufzunehmen, wobei die zweite Position relativ zu der ersten Position veränderlich ist, ohne die gesamte Vorrichtung zu bewegen. Dies kann beispielsweise, wie oben dargestellt, durch ein Absaugelement erzielt werden, welches ein Gelenk aufweist, oder einen flexiblen Bereich aufweist, so dass das Absaugelement verformt werden kann. Alternativ oder zusätzlich kann das Abgabeelement ein Gelenk aufweisen, oder einen flexiblen Bereich aufweisen. Weiterhin kann das Abgabeelement eine bewegliche Abgabeöffnung aufweisen, um eine Flüssigkeit in veränderlicher Richtung aus der Vorrichtung abzugeben. Das Absaugelement kann eine bewegliche Absaugöffnung aufweisen, um eine Flüssigkeit in veränderlicher Richtung von einem Patienten abzuführen.

In einer Ausführungsform ist die Vorrichtung ausgebildet und eingerichtet, eine Flüssigkeit an einer tiefsten Stelle eines zu behandelnden Bereichs eines Patientengewebes aufzunehmen. Hierbei kann die Vorrichtung insbesondere derart ausgestaltet sein, dass das Absaugelement, und insbesondere eine Absaugöffnung des Absaugelements, an einer entsprechend hervorstehenden Position der Vorrichtung angebracht sind. Insbesondere kann die Absaugöffnung an einer Position angeordnet sein, die innerhalb der Vorrichtung am weitesten von dem Behälter entfernt ist.

In einer Ausführungsform kann die Abgabeöffnung einen Durchmesser von 0,5 mm bis 3 mm aufweisen. In einer Ausführungsform kann die Absaugöffnung einen Durchmesser von 0,5 mm bis 3 mm aufweisen. In einer Ausführungsform kann die Absaugöffnung einen Durchmesser aufweisen, der mindestens gleich groß oder größer als der Durchmesser der Abgabeöffnung ist.

In einer Ausführungsform weist eine erfindungsgemäße Vorrichtung einen Behälter, einen Adapter, ein Abgabeelement, ein erstes Verbindungselement, ein zweites Verbindungselement, ein drittes Verbindungselement, ein viertes Verbindungselement, eine erste Durchführung und eine zweite Durchführung auf, wobei der Behälter das erste Verbindungselement aufweist, wobei der Adapter das zweite Verbindungselement, das dritte Verbindungselement und die erste Durchführung zur Abgabe einer Flüssigkeit aus dem Behälter aufweist, wobei das Abgabeelement das vierte Verbindungselement und die zweite Durchführung zur Abgabe einer Flüssigkeit aus der Vorrichtung aufweist.

Das dritte Verbindungselement und/oder das vierte Verbindungselement können bevorzugt ein Gewinde aufweisen. Das dritte Verbindungselement ist bevorzugt ausgebildet und eingerichtet, mit dem vierten Verbindungselement eine formschlüssige Verbindung auszubilden. Die formschlüssige Verbindung ist bevorzugt eine druckfeste fluidleitende Verbindung.

In einer Ausführungsform ist das dritte Verbindungselement an einer Außenseite des Adapters angeordnet. In einer Ausführungsform ist das vierte Verbindungselement an einer Innenseite des Abgabeelements angeordnet. Der Begriff "Innenseite" bezeichnet hierbei eine Oberfläche, welche in Richtung der Mittelachse des Abgabeelements weist.

In einer Ausführungsform ist das dritte Verbindungselement als Außengewinde ausgebildet, und das vierte Verbindungselement ist als Innengewinde ausgebildet. Das dritte und das vierte Verbindungselement können gemeinsam miteinander einen Bajonettverschluss bilden.

Die Vorrichtung ist in einer Ausführungsform ausgebildet und eingerichtet, durch Eingreifen des ersten Verbindungselements in das zweite Verbindungselement und durch Eingreifen des dritten Verbindungselements in das vierte Verbindungselement eine druckfeste fluidleitende Verbindung zwischen dem Behälter, dem Adapter und dem Abgabeelement herzustellen.

In einer Ausführungsform ist die Vorrichtung ausgebildet und eingerichtet, eine Flüssigkeit durch Zusammendrücken des Behälters mit einem Druck zu beaufschlagen, und hierdurch die Flüssigkeit über die erste Durchführung des Adapters und die zweite Durchführung des Abgabeelements aus der Vorrichtung an einen Patienten abzugeben.

In einer Ausführungsform weist das Abgabeelement eine Austrittsöffnung auf. In einer Ausführungsform weist das Abgabeelement mehrere Austrittsöffnungen auf. Die mehreren Austrittsöffnungen können bevorzugt in verschiedene Richtungen weisen. In einer Ausführungsform kann eine Abgabeöffnung einen Durchmesser von 0,5 mm bis 3 mm aufweisen.

In einer Ausführungsform weist das Absaugelement eine Absaugöffnung auf. In einer Ausführungsform weist die Austrittsöffnung eine Querschnittsfläche auf, die kleiner oder gleich groß als/wie die Querschnittsfläche der Absaugöffnung ist. Hierdurch kann erreicht werden, dass die aus der Vorrichtung abgegebene Flüssigkeit und die darin gelösten oder suspendierten Verunreinigungen möglichst vollständig abgeführt werden können.

In einigen Ausführungsformen ist die Vorrichtung eingerichtet, ein Verhältnis der Fördermengen von Absaugelement zu Abgabeelement von mindestens 1 zu erreichen. Dies bedeutet, dass die Vorrichtung ausgebildet und eingerichtet ist, gleichzeitig mindestens genauso viel Flüssigkeit über das Absaugelement abzuführen, wie über das Abgabeelement abgegeben wird. Hierdurch kann besser sichergestellt werden, dass gelöste Biofilme und abgelöstes (d.h. debridiertes) infiziertes Gewebe wirkungsvoll von dem Patienten entfernt werden können. In einigen Ausführungsformen beträgt das Verhältnis der Fördermengen von Absaugelement zu Abgabeelement mindestens 1,0; 1,2; 1,5; oder mindestens 2. In diesem Zusammenhang kann es weiterhin vorteilhaft sein, wenn der Innendurchmesser des Absaugelements im Vergleich zum Innendurchmesser des Abgabeelements entsprechend größer dimensioniert ist.

In einer Ausführungsform ist die Absaugöffnung in einem Abstand von höchstens 4 cm, bevorzugt höchstens 3 cm, höchstens 2 cm oder höchstens 1 cm zu der Austrittsöffnung angeordnet.

Das Absaugelement und/oder das Abgabeelement können einen gekrümmten Bereich aufweisen. Das Absaugelement und/oder das Abgabeelement können insbesondere einen gekrümmten Leitungsbereich aufweisen, wie hierin beschrieben. Dies ist insbesondere vorteilhaft, wenn das Absaugelement und das Abgabeelement gemeinsam ein Stielelement bilden. Hierdurch können beispielsweise Bereiche der posterioren Kondylen leichter erreicht und behandelt werden. Demnach weist die Vorrichtung in einer Ausführungsform ein Stielelement mit einem gekrümmten Leitungsbereich auf, wobei das Absaugelement und das Abgabeelement in dem Stielelement angeordnet sind.

In einer Ausführungsform ist die Austrittsöffnung in einem Abstand von höchstens 8 mm, bevorzugt höchstens 7 mm, höchstens 6 mm oder höchstens 5 mm zu dem vierten Verbindungselement angeordnet.

In einer Ausführungsform ist die Absaugöffnung in einem Abstand von höchstens 8 mm, bevorzugt höchstens 7 mm, höchstens 6 mm oder höchstens 5 mm zu dem vierten Verbindungselement angeordnet.

Durch diese Nähe der Austrittsöffnung bzw. Absaugöffnung zum vierten Verbindungselement kann insbesondere in Verbindung mit einem gekrümmten Abgabeelement bzw. Absaugelement erreicht werden, dass die Austrittsöffnung bzw. Absaugöffnung besser durch den Spalt zwischen der Femurkomponente und der Tibiakomponente eines Knie-Implantats gelangen können.

In einer Ausführungsform weist die Vorrichtung ein Abgabeelement mit einem endständig an dem Abgabeelement angeordneten Bürstenkopf mit einer Vielzahl von Borsten auf.

In einer Ausführungsform weist die Vorrichtung ein Absaugelement auf, und die Borsten weisen eine geeignete Größe auf, um durch das Absaugelement abführbar zu sein. Hierdurch können gegebenenfalls von der Vorrichtung abgelöste Borsten aus einer behandelten Wunde entfernt werden.

Die Vorrichtung kann ein Absaugelement zur Abführung einer Flüssigkeit und ein Abgabeelement aufweisen, wobei das Absaugelement in unmittelbarer Verbindung mit dem Abgabeelement angeordnet ist. Hierbei kann das Absaugelement gemeinsam mit dem Abgabeelement ein Stielelement bilden. Das Absaugelement und das Abgabeelement können innerhalb dieses Stielelements jeweils Durchführungen aufweisen, die innerhalb des Stielelements abschnittsweise parallel angeordnet sein können. Das Stielelement kann einen flexiblen oder gekrümmten Leitungsbereich aufweisen. Dies kann es einem Benutzer ermöglichen, schwer zugängliche Stellen eines Patienten mit der Vorrichtung zu behandeln. Die Vorrichtung kann ein Abgabeelement mit einer Austrittsöffnung und ein Absaugelement mit einer Absaugöffnung aufweisen, wobei die Austrittsöffnung eine Querschnittsfläche aufweist, die kleiner oder gleich groß als/wie die Querschnittsfläche der Absaugöffnung ist.

Der Bürstenkopf kann ein oder mehrere Austrittsöffnungen aufweisen, die zwischen den Borsten angeordnet ist/sind. Die Austrittsöffnungen sind bevorzugt zur Abgabe einer Flüssigkeit aus dem Behälter ausgebildet und eingerichtet. Die Austrittsöffnungen sind bevorzugt fluidleitend mit dem Behälter verbunden. Die Austrittsöffnungen können durch die hierin beschriebene erste Durchführung und die hierin beschriebene zweite Durchführung mit dem Behälter fluidleitend verbunden sein.

In einer Ausführungsform sind die Borsten eingefärbt, um einen optischen Kontrast zu Blut und Körpergewebe zu bilden. Bevorzugt weisen die Borsten einen Farbstoff auf. Der Farbstoff weist bevorzugt ein absolutes Emissionsmaximum im Bereich von 490 nm bis 575 nm auf.

In einer Ausführungsform weisen die Borsten eine Länge von 1,5 bis 20 mm auf. In einer Ausführungsform weisen die Borsten eine Länge von 5 bis 15 mm auf. In einer Ausführungsform weisen die Borsten einen Durchmesser von mindestens 1 mm auf.

In einer Ausführungsform ist die Vorrichtung ausgebildet und eingerichtet, eine Flüssigkeit aus dem Behälter an oder benachbart zu dem Bürstenkopf abzugeben, und gleichzeitig eine Flüssigkeit von oder benachbart zu dem Bürstenkopf abzuführen. Dies ermöglicht es einem Benutzer der Vorrichtung, eine Flüssigkeit aus der Vorrichtung an einem Zielort abzugeben, insbesondere an eine chirurgische Wunde, und mithilfe der Borsten und der abgegebenen Flüssigkeit mikrobiell belastetes Gewebe abzutragen. Das abgetragene Gewebe kann gleichzeitig mithilfe eines Absaugelement der Vorrichtung abgeführt werden. Hierdurch können infiziertes Gewebe und Biofilme aus einem Patienten entfernt werden, sodass nicht die Gefahr besteht, dass Krankheitserreger im Patienten verbleiben oder verteilt werden. Beispielsweise kann auf diese Weise ein Wiederanhaften von Bakterien aus Biofilmen an ein Implantat oder Gelenk vermieden werden.

In einer Ausführungsform ist die Vorrichtung ausgebildet und eingerichtet, eine Flüssigkeit aus dem Behälter an ein Gewebe eines Patienten abzugeben, und das Gewebe des Patienten mithilfe des Bürstenkopfs zu reinigen.

Die Borsten sind bevorzugt aus einem biokompatiblen Polymer gebildet, zum Beispiel Polyethylen, Polypropylen, Polyamid oder ähnliches.

Der Bürstenkopf kann Borsten mit unterschiedlicher Länge aufweisen, um die Reinigung von Hinterschneidungen zu erleichtern. Beispielsweise können die unterschiedlich langen Borsten bogenförmig auf dem Bürstenkopf angeordnet sein. Zum Beispiel können die Borsten im distalen Bereich des Bürstenkopfs länger sein als die Borsten am proximalen Bereich des Bürstenkopfs. Der distale Bereich des Bürstenkopfs ist diejenige Seite, die am weitesten vom Adapter entfernt angeordnet ist. Der proximale Bereich des Bürstenkopfs ist diejenige Seite, die in Richtung des Adapters weist.

In einigen Ausführungsformen weist die Vorrichtung eine Vielzahl von Austrittsöffnungen zur Abgabe einer Flüssigkeit aus dem Behälter auf. Die Austrittsöffnungen können am Bürstenkopf in der Nähe der Borsten, oder an den Borsten selbst angeordnet sein, oder beides. Die Borsten können hohl sein, um den Transport einer Flüssigkeit zu ermöglichen. Die Vorrichtung kann eingerichtet sein, eine Flüssigkeit aus dem Abgabeelement durch Innenräume der einzelnen Borsten hindurchzuleiten, und an der Spitze der Borsten freizusetzen.

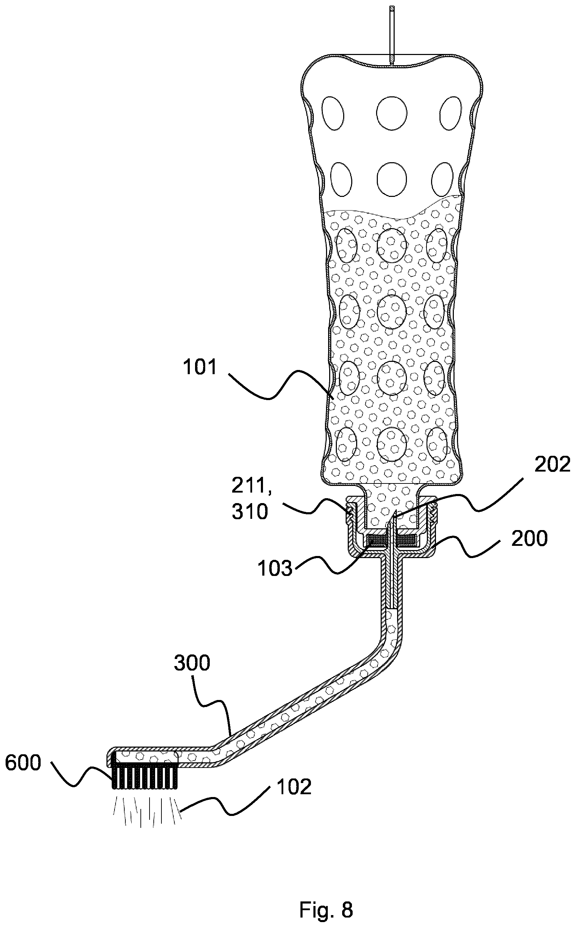

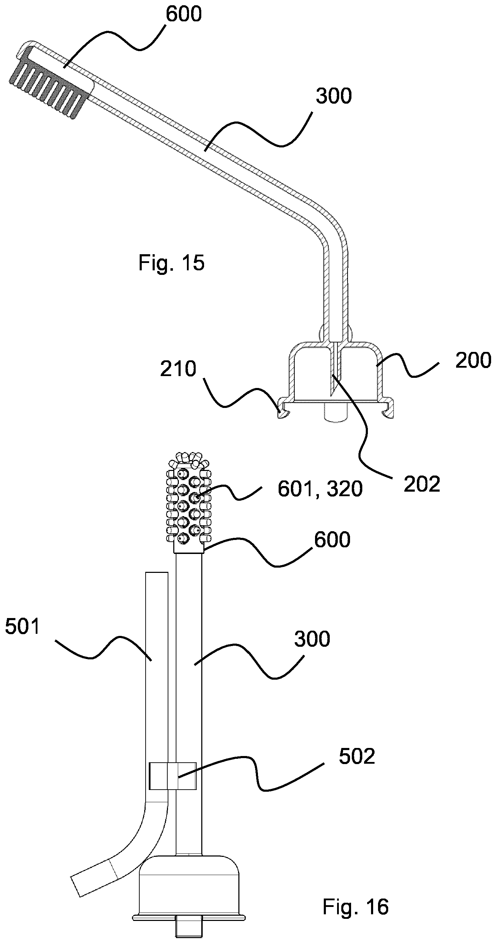

In einer Ausführungsform weist das Abgabeelement einen rohrförmigen Bürstenkopf auf, an welchem mehrere Austrittsöffnungen umlaufend angeordnet sind. Weiterhin kann der rohrförmige Bürstenkopf umlaufend angeordnete Borsten aufweisen. Die Absaugöffnung des Absaugelements kann hierbei in räumlicher Nähe zu den Austrittsöffnungen angeordnet sein. Solche Ausführungsformen können beispielsweise besonders vorteilhaft zur Behandlung von Femurkanälen sein. Die Borsten können passend zu anatomischen Gegebenheiten des gewünschten Einsatzorts in verschiedener Weise angeordnet sein. In einer Ausführungsform erstrecken sich die Borsten ausgehend von dem Bürstenkopf in Richtung des Behälters. Dies bedeutet, dass die Spitzen der Borsten in Richtung des Behälters weisen. Die Borsten weisen hierbei somit in Bezug auf die Vorrichtung gewissermaßen nach innen, wie beispielhaft in Figur 15 gezeigt. In einer Ausführungsform erstrecken sich die Borsten ausgehend von dem Bürstenkopf in Gegenrichtung des Behälters. Die Borsten weisen hierbei somit in Bezug auf die Vorrichtung nach außen, wie beispielhaft in Figur 8 gezeigt. In einer Ausführungsform sind die Borsten umlaufend an dem Bürstenkopf angeordnet. Die Borsten sind hierbei somit nach Art einer Rundbürste an dem Bürstenkopf angeordnet, wie beispielhaft in Figur 16 gezeigt.

In einigen Ausführungsformen umfasst der Bürstenkopf einen gewinkelt angeordneten Ausstromkanal. Der Ausstromkanal kann in Bezug auf die Ausrichtung der Borsten in einem Winkel von mehr als 15°, zum Beispiel ungefähr 45°, angeordnet sein. Hierdurch kann eine Flüssigkeit, zum Beispiel in Form von Sprühstößen oder als kontinuierlicher Strahl einer Flüssigkeit, in den Bereich neben dem Bürstenkopf abgegeben werden, um die Reinigungsleistung der Bürste zu verbessern. Die Flüssigkeit kann an einem äußeren Ende des Ausstromkanals über eine Ausstromöffnung abgegeben werden.

In einer Ausführungsform weist die Vorrichtung ein Abgabeelement auf, welches einen schalenförmigen Trichter aufweist. Ein solcher Trichter kann ausgebildet und eingerichtet sein, einen Bereich zu definieren, an welchen eine Flüssigkeit aus der Vorrichtung abgegeben wird. Der Trichter kann ein Volumenelement begrenzen, an welches eine Flüssigkeit aus der Vorrichtung ausbringbar ist.

Der Trichter kann ein Randelement aufweisen. Das Randelement ist bevorzugt ausgebildet und eingerichtet, mit einer Gewebeoberfläche eines Patienten abzuschließen, und dadurch einen geschlossenen Innenraum des Trichters zu bilden. Das Randelement kann als gummielastische Dichtlippe ausgebildet sein. Das Randelement ist bevorzugt randständig an dem Trichter angeordnet.

Der Trichter weist bevorzugt eine Mittelachse auf. Die Mittelachse kann parallel zu der zweiten Durchführung, d. h. der Durchführung des Abgabeelements, angeordnet sein. Die Mittelachse kann der Fließrichtung einer Flüssigkeit entsprechen, die aus der Vorrichtung abgegeben wird. In einer Ausführungsform ist die Vorrichtung ausgebildet und eingerichtet, eine Flüssigkeit an verschiedenen Positionen innerhalb des Trichters abzugeben. In einer Ausführungsform ist die Vorrichtung ausgebildet und eingerichtet, eine Flüssigkeit in verschiedenen Richtungen im Verhältnis zur Mittelachse des Trichters abzugeben. Bevorzugt liegen die verschiedenen Positionen innerhalb eines geschlossenen Innenraums des Trichters, der wie oben dargelegt durch den Trichter definiert wird, wenn ein Randelement des Trichters mit einer Gewebeoberfläche eines Patienten abschließt. Der Begriff "geschlossener Innenraum", wie hierin im Zusammenhang mit dem Trichter verwendet, schließt das Vorhandensein des Abgabeelements, einer Belüftungsöffnung und/oder eines Absaugelement nicht aus. Der Begriff "geschlossener Innenraum" ist hierin so definiert, dass der Trichter gemeinsam mit einer Gewebeoberfläche eines Patienten einen im Wesentlichen abgetrennten Raum bildet, innerhalb dessen eine Flüssigkeit aus der Vorrichtung abgegeben und/oder aus dem eine Flüssigkeit abgeführt werden kann. In einer Ausführungsform ist die Vorrichtung ausgebildet und eingerichtet, eine Flüssigkeit an jegliche Positionen einer Gewebeoberfläche, die durch Inkontaktbringen des Trichters mit einem Patientengewebe definiert werden, abzugeben, bevorzugt ohne die gesamte Vorrichtung translatorisch zu bewegen.

Der Trichter kann ein gummielastisches Material aufweisen, oder aus einem gummielastischen Material bestehen. Hierdurch kann erreicht werden, dass der Trichter durch einen Benutzer manuell elastisch verformbar ist. Durch eine solche Verformung kann die Abgaberichtung der Flüssigkeit gesteuert werden. Hierdurch kann, wie oben dargestellt, die Flüssigkeit an verschiedene Positionen abgegeben werden, ohne die Vorrichtung als Ganzes translatorisch zu bewegen.

In einer Ausführungsform weist der Trichter ein Gelenk auf. In ähnlicher Weise wie oben dargestellt, kann ein solches Gelenk zur Steuerung der Abgaberichtung eine Flüssigkeit dienen, welche aus der Vorrichtung abgegeben werden kann. Das Gelenk kann beispielsweise als flexibler Bereich des Trichters ausgebildet sein. Ein solcher Bereich kann ein Material aufweisen, das eine höhere Elastizität und/oder höhere Verformbarkeit als das umgebende Material des Trichters aufweist. Alternativ oder zusätzlich kann ein solcher Bereich eine geringere Dicke, d. h. Materialstärke, im Vergleich zu den angrenzenden Teilen des Trichters aufweisen.

Das Gelenk kann vorteilhafterweise benachbart zu dem Adapter angeordnet sein. Das Gelenk kann an einem Halsbereich des Trichters angeordnet sein. Das Gelenk kann benachbart zu dem vierten Verbindungselement, d. h. dem Verbindungselement des Abgabeelements, welches zur Verbindung mit dem Adapter dient, angeordnet sein.

Der Trichter kann drehbar an der Vorrichtung angeordnet sein. Bevorzugt kann der Trichter drehbar um eine Achse angeordnet sein, welche parallel zu einer Längsachse des Behälters, eine Längsachse der ersten Durchführung und/oder einer Längsachse der zweiten Durchführung ist.

Der Trichter kann eine Trichterwand aufweisen, welche das Abgabeelement umgibt.

In einer Ausführungsform weist die Vorrichtung ein Absaugelement zur Abführung einer Flüssigkeit auf. Das Absaugelement kann bevorzugt an dem Abgabeelement angeordnet sein. Das Absaugelement kann eine Absaugöffnung aufweisen. Die Absaugöffnung kann zur Aufnahme einer Flüssigkeit ausgebildet und eingerichtet sein. Der Trichter kann eine Wand aufweisen. Die Absaugöffnung kann in der Wand des Trichters angeordnet sein.

Der Trichter kann weiterhin eine Belüftungsöffnung aufweisen. Die Belüftungsöffnung kann in einer Wand des Trichters angeordnet sein. In einem Nutzungsszenario der Vorrichtung, in welchem ein Randelement des Trichters mit einer Gewebeoberfläche eines Patienten abschließt, und somit wie oben dargestellt einen geschlossenen Innenraum des Trichters definiert, kann eine solche Belüftungsöffnung zum Druckausgleich für das Absaugelement dienen.

Die Belüftungsöffnung ist bevorzugt an einer gegenüberliegenden Seite des Trichters angeordnet, an welcher sich die Absaugöffnung befindet. Bei einem Trichter mit im Wesentlichen kreisförmigem Abschlussrand kann dies bevorzugt einen Winkel von 180° zwischen der Belüftungsöffnung und der Absaugöffnung sein. Dies gewährleistet einen ausreichenden Abstand zwischen der Belüftungsöffnung und der Absaugöffnung. Wenn die Belüftungsöffnung dagegen in unmittelbarer Nähe der Absaugöffnung angeordnet ist, könnte eine wirksame Abführung der aus dem Trichter abzuführenden Flüssigkeit behindert werden, da in diesem Fall Luft direkt aus der Belüftungsöffnung in die Absaugöffnung strömen könnte, ohne auf dem Weg dazwischen eine Sogwirkung auf die Flüssigkeit auszuüben. Die Absaugöffnung kann in unmittelbarer Nähe zu einer Dichtlippe des Trichters angeordnet sein.

Das Abgabeelement kann eine stielförmige Düse aufweisen. Die stielförmige Düse kann fluidleitend mit der zweiten Durchführung verbunden sein. Die Düse kann schwenkbar um die Mittelachse des Trichters sein. Dies ermöglicht es, eine Flüssigkeit aus der Vorrichtung an verschiedene Positionen innerhalb eines Innenraums des Trichters abzugeben.

Die stielförmige Düse ist bevorzugt ausgebildet und angeordnet, einen direkten Kontakt der Düse mit einem Gewebe eines Patienten zu verhindern. Hierzu kann die Düse innerhalb des Trichters zurückgesetzt angeordnet sein, sodass sie sich ausgehend von dem Adapter oder Behälter nicht auf die volle Distanz erstreckt, an der sich das gegenüberliegende Ende des Trichters, beispielsweise der Rand, und insbesondere eine Dichtlippe des Trichters, befindet.

In einer Ausführungsform ist das Abgabeelement, insbesondere die stielförmige Düse, eingefärbt. Dies kann es einem Benutzer erleichtern, die Position und/oder Orientierung des Abgabeelements innerhalb des Trichters zu verfolgen. Bevorzugt weist das Abgabeelement hierzu einen Farbstoff auf. Der Farbstoff weist bevorzugt ein absolutes Emissionsmaximum im Bereich von 490 nm bis 575 nm auf. Ein eingefärbte Abgabeelement kann insbesondere in Ausführungsformen von Vorteil sein, in welchen der Trichter transluzent oder transparent ist.

Die Düse des Adapters ist bevorzugt mit der Düse des Abgabeelements verbindbar. In einer Ausführungsform ist die Düse des Adapters formschlüssig mit der Düse des Abgabeelements verbindbar. In einer Ausführungsform ist die Düse des Adapters lösbar mit der Düse des Abgabeelements verbindbar. In einer Ausführungsform ist die Düse des Adapters in die Düse des Abgabeelements einführbar.

Ein weiterer Aspekt der Erfindung betrifft ein Verfahren zur Abgabe einer Flüssigkeit mithilfe einer hierin beschriebenen Vorrichtung. Das Verfahren kann mithilfe jeder der hierin beschriebenen Vorrichtungen durchgeführt werden.

Die Vorrichtung kann einen Behälter, einen Adapter und ein Abgabeelement aufweisen. Der Behälter ist bevorzugt elastisch verformbar. Der Behälter ist bevorzugt zur Aufnahme einer Flüssigkeit ausgebildet und eingerichtet. Der Behälter kann eine Flüssigkeit enthalten. Der Adapter kann mit dem Behälter fluidleitend verbindbar sein. Das Abgabeelement kann zur Abgabe einer Flüssigkeit aus der Vorrichtung ausgebildet und eingerichtet sein. Der Behälter kann ein erstes Verbindungselement aufweisen. Der Adapter kann ein zweites Verbindungselement aufweisen. Der Adapter kann ein drittes Verbindungselement aufweisen. Der Adapter kann eine erste Durchführung zur Abgabe einer Flüssigkeit aus dem Behälter aufweisen. Das Abgabeelement kann ein viertes Verbindungselement aufweisen. Das Abgabeelement kann eine zweite Durchführung zur Abgabe einer Flüssigkeit aus der Vorrichtung aufweisen.

In einer Ausführungsform ist die Vorrichtung ausgebildet und eingerichtet, durch Eingreifen des ersten Verbindungselements in das zweite Verbindungselement und durch Eingreifen des dritten Verbindungselements in das vierte Verbindungselement eine druckfeste fluidleitende Verbindung zwischen dem Behälter, dem Adapter und dem Abgabeelement herzustellen.

In einer Ausführungsform ist die Vorrichtung ausgebildet und eingerichtet, eine Flüssigkeit durch Zusammendrücken des Behälters mit einem Druck zu beaufschlagen, und hierdurch die Flüssigkeit über die erste Durchführung des Adapters und die zweite Durchführung des Abgabeelements aus der Vorrichtung an einen Patienten abzugeben.

In einer Ausführungsform weist das Verfahren zur Abgabe einer Flüssigkeit die nachfolgenden Schritte auf: Verbinden des Behälters mit dem Adapter unter Ausbildung einer druckfesten fluidleitenden Verbindung zwischen dem Behälter und dem Adapter; Verbinden des Adapters mit dem Abgabeelement unter Ausbildung einer druckfesten fluidleitenden Verbindung zwischen dem Adapter und dem Abgabeelement; und Druck-beaufschlagen einer Flüssigkeit innerhalb des Behälters, um die Flüssigkeit über den Adapter und das Abgabeelement aus der Vorrichtung abzugeben. Das Druck-beaufschlagen der Flüssigkeit kann hierbei bevorzugt durch Zusammendrücken des Behälters erfolgen. Das Verfahren kann weiterhin das Abführen einer Flüssigkeit mithilfe eines hierin beschriebenen Absaugelements umfassen. Das Verfahren kann den Anschluss des Absaugelement an eine externe Vakuumquelle umfassen. Das Verbinden des Adapters mit dem Behälter kann ein Eingreifen des ersten Verbindungselements in das zweite Verbindungselement umfassen. Die Ausbildung einer druckfesten fluidleitenden Verbindung zwischen dem Adapter und dem Behälter kann das Durchstechen eines Septums mithilfe eines Hohldorns umfassen, wie hierin beschrieben. In einer Ausführungsform wird das Verfahren *in vitro* durchgeführt. In einer Ausführungsform umfasst das Verfahren ein Inkontaktbringen einer Flüssigkeit aus der Vorrichtung mit einem Patienten.

Ein weiterer Aspekt betrifft ein Therapieverfahren, in welchem eine hierin beschriebene Vorrichtung zur Entfernung von Biofilmen oder infiziertem Gewebe eingesetzt wird. Hierbei werden Biofilme oder infiziertes Gewebe mithilfe der aus der Vorrichtung abgegebenen Flüssigkeit gelöst bzw. suspendiert. Das Therapieverfahren kann jeden der oben beschriebenen Schritte und Merkmale des Verfahrens zur Abgabe einer Flüssigkeit umfassen.

Ein weiterer Aspekt betrifft einen Wirkstoff zur Verwendung in der Behandlung einer chirurgischen Wunde, insbesondere zur Desinfektion einer chirurgischen Wunde, wobei der Wirkstoff mithilfe einer hierin beschriebenen Vorrichtung an die Wunde abgegeben wird.

In einer Ausführungsform ist der Wirkstoff ausgewählt aus der Gruppe bestehend aus Chlorhexidinglukonat, Benzalkoniumchlorid, Octenidindihydrochlorid, einem Alkalihypochlorit, einem Erdalkalihypochlorit, und einem Alkalichlorit. In einer Ausführungsform umfasst die Flüssigkeit Natriumdodecylsulfat oder eine physiologische Kochsalzlösung.

In einer Ausführungsform wird der Wirkstoff als wässrige Lösung bereitgestellt, wobei die Lösung ausgewählt ist aus der Gruppe bestehend aus
einer Lösung von Benzalkoniumchlorid mit einer Benzalkoniumchlorid-Konzentration von 50 bis 1500 ppm;
einer Lösung von Chlorhexidindiglukonat und Benzalkoniumchlorid, wobei die Konzentration an Chlorhexidindiglukonat 50 bis 600 ppm beträgt und die Konzentration an Benzalkoniumchlorid 50 bis 250 ppm beträgt;
einer Lösung von Octenidindihydrochlorid mit einer Konzentration von 50 bis 2000 ppm;
einer Lösung eines Alkalihypochlorits mit einer Konzentration an Hypochlorit von 50 bis 2000 ppm;
einer Lösung eines Erdalkalihypochlorits mit einer Konzentration an Hypochlorit 50 bis 2000 ppm;
einer Lösung eines Alkalichlorits mit einer Konzentration an Chlorit von 50 bis 2000 ppm, einer Lösung von Natriumdodecylsulfat mit einer Konzentration an Natriumdodecylsulfat von 50 bis 2000 ppm; und
einer physiologischen Kochsalzlösung mit einer Konzentration an Natriumchlorid von 0,9 Gew.-%.

In einer Ausführungsform umfasst das Verfahren ein Debridement, bevorzugt ein DAIR-Verfahren. In einer Ausführungsform umfasst das Verfahren die Abgabe einer Flüssigkeit während einer Knie- oder Hüftgelenksoperation, insbesondere bei einer Revisions-Operation.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

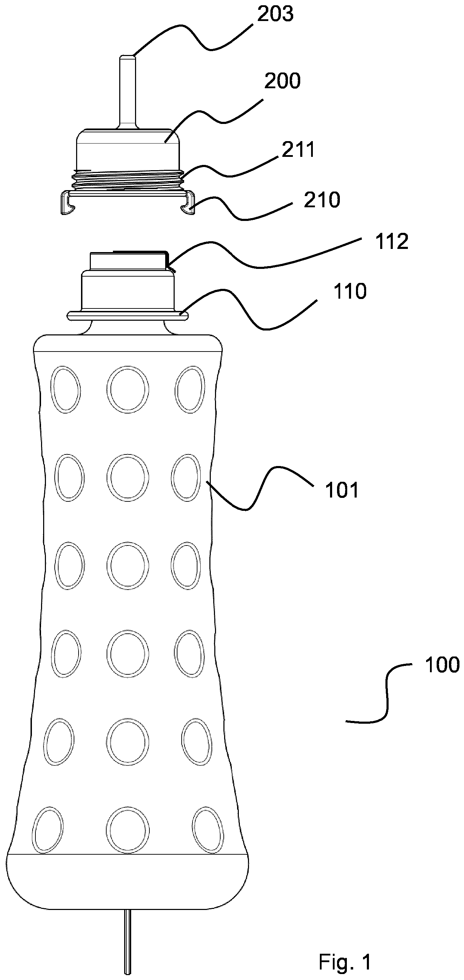

**Figur 1** zeigt beispielhaft eine Ausführungsform einer erfindungsgemäßen Vorrichtung **100.** Die Vorrichtung weist einen Behälter **101** auf, der zur Aufnahme einer Flüssigkeit geeignet ist. Der Behälter kann mit einer Flüssigkeit vorbefüllt sein. Der Behälter ist elastisch verformbar, sodass er mit der Hand eines Benutzers zusammengedrückt werden kann, um eine darin eingefügte Flüssigkeit mit einem Druck beaufschlagen zu können, und dadurch die Flüssigkeit aus der Vorrichtung abgegeben zu können. Im hier gezeigten Beispiel weist der Behälter eine Oberflächenstruktur auf, die mehrere muldenartige kreisförmige Vertiefungen aufweist. Dies kann es einem Benutzer erleichtern, den Behälter sicher zugreifen und festzuhalten. Im hier gezeigten Beispiel ist der Behälter als Kunststoffflasche ausgebildet. Der Behälter weist einen Flaschenhals auf, an dem ein Anschlussstück mit einem ersten Verbindungselement **110** angeordnet ist. Das erste Verbindungselement **110** ist hier als ringförmiger Vorsprung ausgebildet. Der Behälter ist durch ein Siegel **112** verschlossen, welches hier als aufgeklebte oder verschweißte Folie ausgebildet ist, die von einem Benutzer manuell entfernt werden kann.

Die Vorrichtung weist weiterhin einen Adapter **200** auf, der in der hier gezeigten Ausführungsform als separates Teil ausgestaltet ist. Der Adapter **200** weist hier die Form einer Kappe auf, welche das Anschlussstück des Behälters **101** von mehreren Seiten umgibt, und dadurch eine formschlüssige Verbindung zwischen dem Behälter **101** und dem Adapter **200** ausbildet.

Der Adapter **200** ist lösbar mit dem Behälter **101** verbindbar. Hierzu weist der Adapter **201** ein zweites Verbindungselement **210** auf, das mit dem ersten Verbindungselement **110** des Behälters **101** verbindbar ist. Das zweite Verbindungselement **210** ist hier als biegsamer Rasthaken ausgebildet. Es können mehrere solcher Rasthaken an dem Adapter **200** angeordnet sein. Beispielsweise kann der Adapter **200** zwei oder vier gleichartige Rasthaken zur Verbindung mit dem ersten Verbindungselement **110** aufweisen. Das erste Verbindungselement **110** ist an einer ersten Seite des Adapters **200** angeordnet, wobei die erste Seite des Adapters **200** zur Verbindung mit dem Behälter **101** vorgesehen ist. An einer zweiten Seite des Adapters **200** ist eine Düse **203** angeordnet, welche hier eine stielförmige Form aufweist. Die Düse **203** ist zur Abgabe einer Flüssigkeit aus dem Behälter **101** eingerichtet. Zwischen dem ersten Verbindungselement **110** und der Düse **203** ist ein drittes Verbindungselement **211** angeordnet, das zur Verbindung mit einem Abgabeelement (in dieser Zeichnung nicht dargestellt) vorgesehen ist.

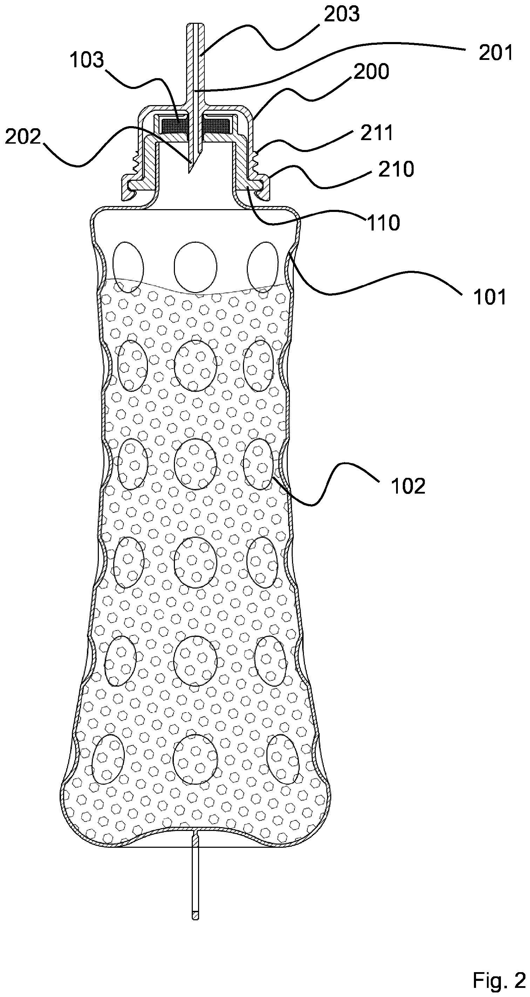

**Figur 2** zeigt eine Querschnittsansicht einer Vorrichtung mit einem Behälter **101,** welcher druckfest und fluidleitend mit einem Adapter **200** verbunden ist. Das erste Verbindungselement **110** ist in das zweite Verbindungselement **210** in Eingriff gebracht, um eine formschlüssige Verbindung zwischen dem Behälter **101** und dem Adapter **200** herzustellen.

Der Behälter **101** ist mit einer Flüssigkeit **102** befüllt. Der Behälter weist weiterhin ein Septum **103** auf, welches ein gummielastisches Material aufweist. Der Adapter **200** weist einen Hohldorn **202** auf, der durch das Septum in einen Innenraum des Behälters **101** eingeführt ist, um eine fluidleitende Verbindung zwischen dem Behälter **101** und dem Adapter **200** auszubilden. Innerhalb des Hohldorns **202** ist eine erste Durchführung **201** angeordnet, um die Flüssigkeit **102** aus dem Behälter **101** durch den Adapter **200** nach außen zu leiten und durch die Düse **203** abzugeben.

Das dritte Verbindungselement **211** bleibt in der hier dargestellten Konfiguration, in welcher der Behälter **101** druckfest und fluidleitend mit dem Adapter **200** verbunden ist, weiterhin zugänglich, um eine Verbindung mit einem Abgabeelement **300** zu ermöglichen.

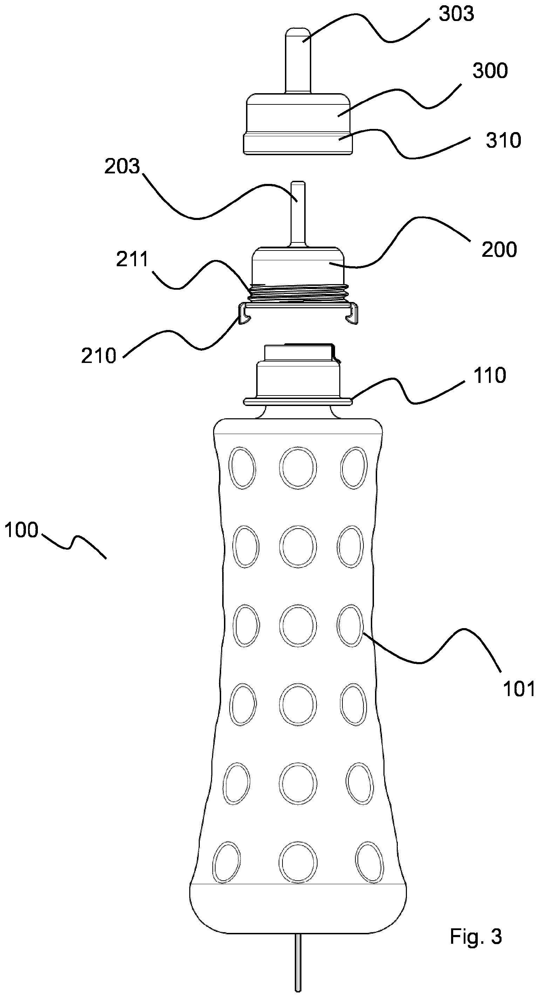

**Figur 3** zeigt eine Querschnittsansicht einer Vorrichtung, welche einen Behälter **101,** einen Adapter **200** und ein Abgabeelement **300** aufweist. Der Behälter **101,** der Adapter **200** und das Abgabeelement **300** sind jeweils als getrennte, miteinander lösbar verbindbare Teile ausgebildet. Der Behälter weist ein erstes Verbindungselement **110** zur Verbindung mit dem Adapter **200** auf. Der Adapter **200** weist ein zweites Verbindungselement **210** zur Verbindung mit dem Behälter **101** auf. Der Adapter **200** weist weiterhin ein drittes Verbindungselement **211** auf, das zur Verbindung mit dem Abgabeelement **300** ausgebildet und eingerichtet ist. Das dritte Verbindungselement **211** ist hier als Außengewinde ausgebildet. Der Adapter **200** weist eine stielförmige Düse **203** auf. Das Abgabeelement **300** weist ein viertes Verbindungselement **310** zur Verbindung mit dem Adapter **200** auf. Das vierte Verbindungselement **310** ist hier als Innengewinde ausgebildet. Das Abgabeelement **300** weist weiterhin eine stielförmige Düse **303** auf. Die Düse **203** ist formschlüssig mit der Düse **303** verbindbar. Hierzu kann die Düse **203** in die Düse **303** eingeführt werden.

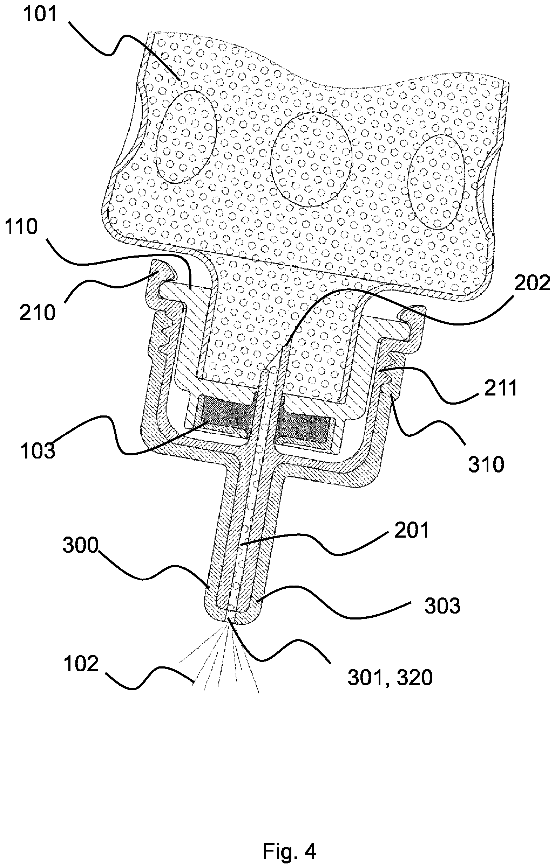

**Figur 4** zeigt einen Ausschnitt einer Querschnittsansicht einer erfindungsgemäßen Vorrichtung, wobei ein Behälter **101** druckfest fluidleitend mit einem Adapter **200** verbunden ist, und der Adapter **200** mit einem Abgabeelement **300** druckfest fluidleitend verbunden ist, um eine Flüssigkeit **102** aus dem Behälter **101** abzugeben. Das erste Verbindungselement **110** des Behälters **101** greift in das zweite Verbindungselement **210** des Adapters ein. Das dritte Verbindungselement **211** des Adapters greift in das vierte Verbindungselement **310** des Abgabeelements **300** sein. Der Hohldorn **202** ist durch das Septum **103** in den Innenraum des Behälters **101** eingeführt. Eine Flüssigkeit **102** kann aus dem Behälter **101** durch eine erste Durchführung **201** des Adapters **200** und eine zweite Durchführung **301** des Abgabeelements **300** nach außen geführt und durch eine Austrittsöffnung **320** des Abgabeelements **300** aus der Vorrichtung an einen Patienten abgegeben werden.

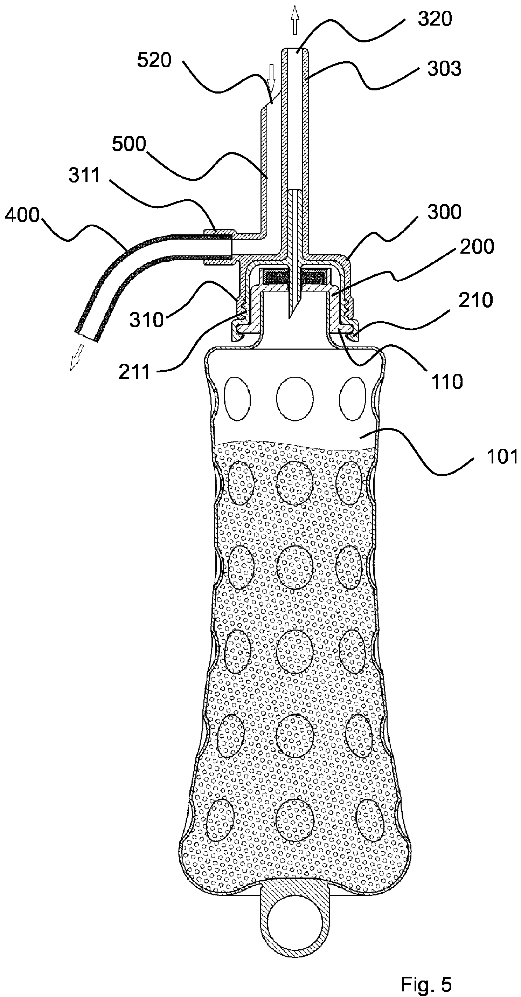

**Figur 5** zeigt einen Ausschnitt einer Querschnittsansicht einer erfindungsgemäßen Vorrichtung, welche weiterhin ein Absaugelement **500** aufweist. Die Vorrichtung weist einen Behälter **101** auf, der über ein erstes Verbindungselement **110** und ein zweites Verbindungselement **210** mit einem Adapter **200** druckfest fluidleitend verbunden ist. Die Vorrichtung weist weiterhin ein Abgabeelement **300** auf, das mithilfe eines dritten Verbindungselements **211** und eines vierten Verbindungselements **310** mit dem Adapter **200** verbunden ist. Das Abgabeelement **300** weist eine Düse **303** mit einer Austrittsöffnung **320** zur Abgabe einer Flüssigkeit **102** aus der Vorrichtung auf. Unmittelbar an dem Abgabeelement ist ein Absaugelement **500** an der Vorrichtung angeordnet. Das Absaugelement **500** weist eine Absaugöffnung **520** auf, wobei die Absaugöffnung **520** benachbart zu der Austrittsöffnung **320** angeordnet ist. Die Absaugöffnung **520** ist zum Abführen einer Flüssigkeit ausgebildet und eingerichtet. Somit kann eine aus dem Behälter abgegebenen Flüssigkeit, die zum Spülen einer chirurgischen Wunde eingesetzt wird, nach Kontakt mit einem Patientengewebe aus dem Bereich der Wunde abgesaugt werden. Die Vorrichtung weist weiterhin ein fünftes Verbindungselement **311** zur Verbindung mit einem Zubehörteil **400** auf. Das Zubehörteil **400** ist hier als Schlauch ausgebildet, welcher an eine externe Vakuumquelle anschließbar ist. Hierdurch kann eine gebrauchte Spülflüssigkeit wirksam abgeführt werden.

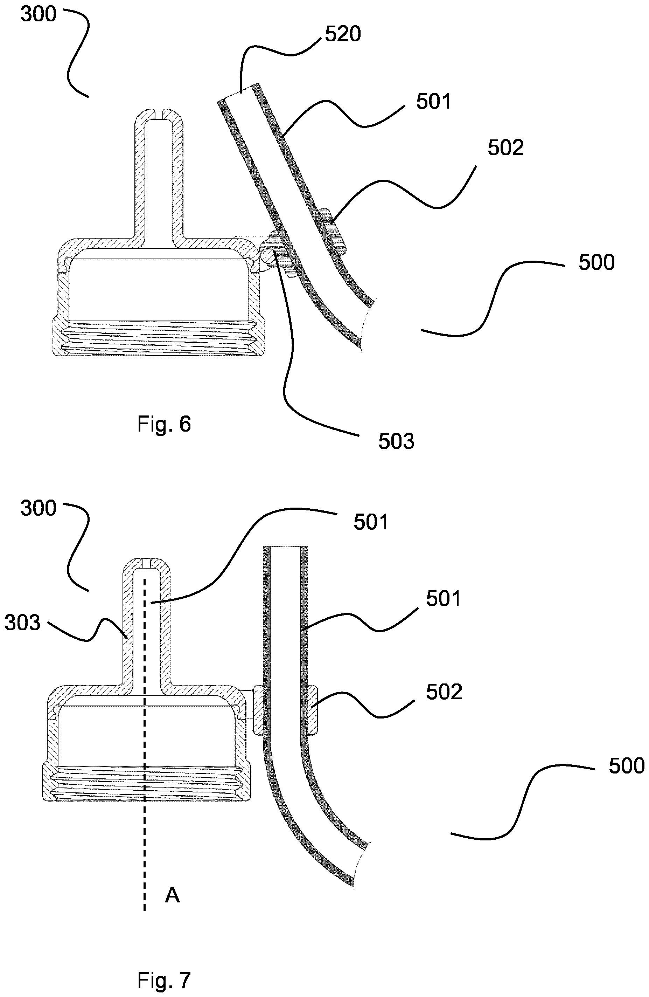

**Figur 6** zeigt eine Querschnittsansicht eines Abgabeelements **300,** welches mit einem Absaugelement **500** verbunden ist. An dem Abgabeelement **300** ist ein Gelenk **503** angeordnet, welches eine Schelle **502** beweglich mit dem Abgabeelement **300** verbindet. Die Schelle **502** ist zum Halten eines Absaugelements **500** ausgebildet und eingerichtet, welches einen Schlauch **501** aufweist. Die Schelle **502** umgibt den Schlauch **501,** und bildet eine kraftschlüssige Verbindung mit diesem aus.

**Figur 7** zeigt eine Querschnittsansicht eines Abgabeelements **300,** welches mit einem Absaugelement **500** verbunden ist. Das Abgabeelement **300** ist mit einer Schelle **502** verbunden, welche einen Schlauch **501** hält, welcher Teil eines Abgabeelements **500** ist. In einigen Ausführungsformen ist die Schelle **502** drehbar um eine Längsachse A des Abgabeelements **300** angeordnet. Die Längsachse A ist hier die Längsmittelachse einer stielförmigen Düse **303** des Abgabeelements **300.** Die Längsachse A verläuft parallel zur Flussrichtung einer Flüssigkeit, die aus dem Behälter über das Abgabeelement **300** nach außen abgegeben wird. Die Schelle **502** kann hierzu beispielsweise an einem drehbaren Ring befestigt sein, welcher drehbar an dem Abgabeelement **300** angeordnet ist. Die Vorrichtung kann ausgebildet und eingerichtet sein, die Schelle **502** aufgrund von Schwerkrafteinwirkung selbsttätig an einem tiefsten Punkt des Adapterelements **200** anzuordnen. Hierzu kann es zweckmäßig sein, einen drehbaren Ring, oder ein vergleichbares Halteelement, welches zur drehbaren Lagerung der Schelle **502** ausgebildet und eingerichtet ist, entsprechend leichtgängig gelagert auszugestalten.

**Figur 8** zeigt eine Querschnittsansicht einer Vorrichtung, welche einen Behälter **100** und ein damit druckfest fluidleitend verbundenes Abgabeelement **300** mit einem Bürstenkopf **600** aufweist. Der Behälter **100** ist hierbei über einen dazwischenliegenden Adapter **200** mit dem Abgabeelement **300** verbunden. Das erste Verbindungselement **110** und das zweite Verbindungselement **210** sind in dieser Zeichnung nicht dargestellt, können aber vorhanden sein. Der Adapter **200** weist ein drittes Verbindungselement **211** auf, welches hier als Außengewinde ausgebildet ist. Das Abgabeelement **300** weist ein viertes Verbindungselement **310** zur Verbindung mit dem dritten Verbindungselement **211** auf. Das vierte Verbindungselement **310** ist hier als Innengewinde ausgebildet. Der Adapter **200** weist einen Hohldorn **202** auf, um ein Septum **103** des Behälters zu durchdringen, und eine fluidleitende Verbindung zwischen dem Behälter **100,** dem Adapter **200** und dem Abgabeelement **300** zu bilden. Die Vorrichtung ist ausgebildet und eingerichtet, eine Flüssigkeit **102** aus dem Behälter **101** über den Adapter **200** und das Abgabeelement **300** im Bereich des Bürstenkopfs **600** aus der Vorrichtung abzugeben. Hierdurch kann eine medizinische Spülflüssigkeit im Bereich des Bürstenkopfs freigesetzt werden, um unter Einwirkung dieser Spülflüssigkeit und gleichzeitiger mechanischer Einwirkung der Borsten des Bürstenkopfs **600** ein Patientengewebe zu behandeln.

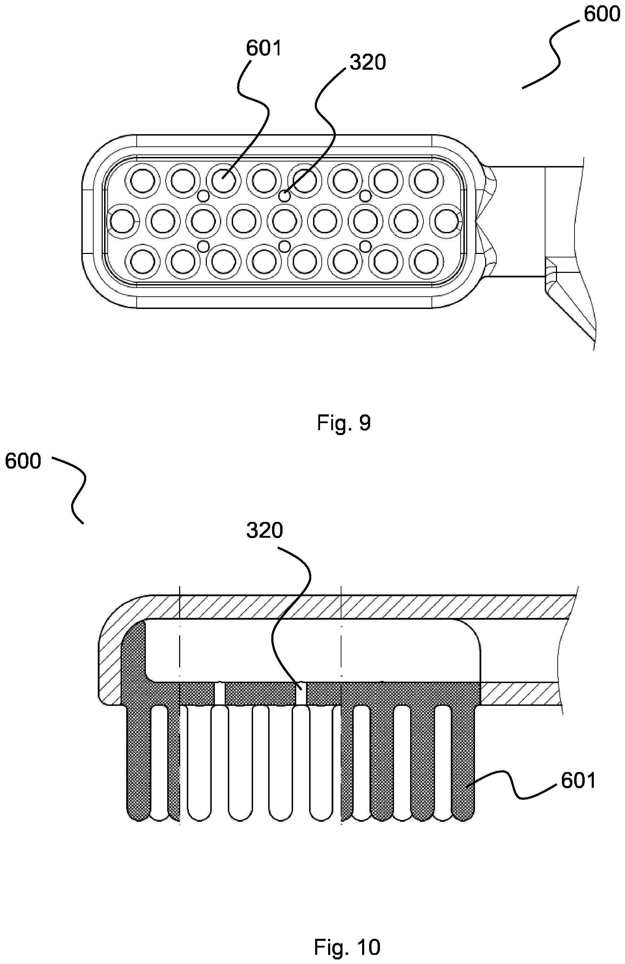

**Figur 9** zeigt einen Ausschnitt eines Abgabeelements **300** mit einem Bürstenkopf **600.** Der Bürstenkopf **600** weist Borsten **601** und dazwischenliegenden angeordnete Austrittsöffnungen **320** zur Abgabe einer Flüssigkeit aus der Vorrichtung auf. Im hier gezeigten Beispiel sind mehrere Austrittsöffnungen **320** im Wesentlichen gleich verteilt, d. h. mit untereinander jeweils vergleichbaren Abständen zueinander, an dem Bürstenkopf **600** angeordnet.

**Figur 10** zeigt einen Ausschnitt eines Abgabeelements **300** mit einem Bürstenkopf **600** als Querschnittsansicht. Mehrere Austrittsöffnungen **320** sind zwischen den Borsten **601** angeordnet, um eine Flüssigkeit aus der Vorrichtung abzugeben.

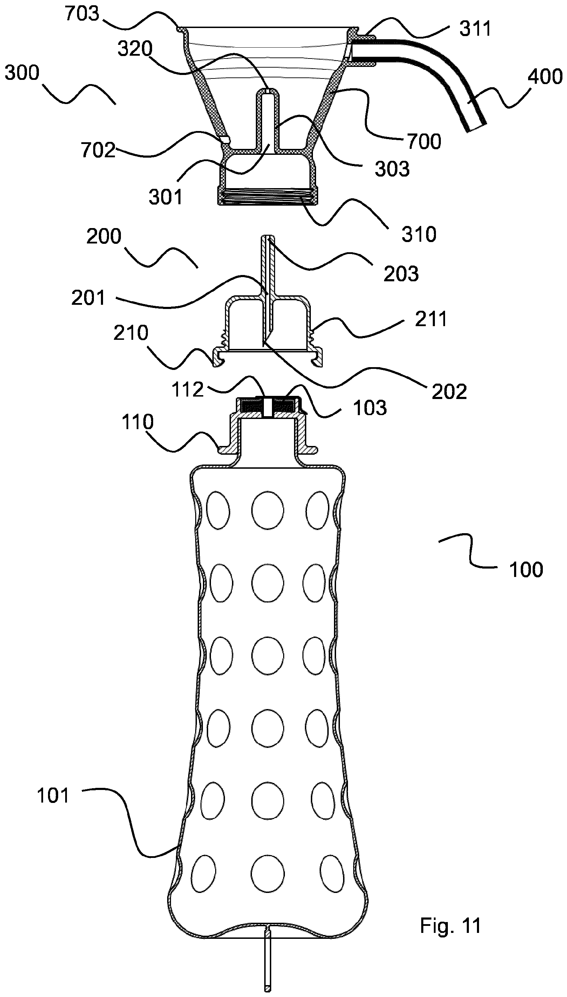

**Figur 11** zeigt eine erfindungsgemäße Vorrichtung **100** mit einem Behälter **101,** einem Adapter **200** und einem Abgabeelement **300.** Das Abgabeelement **300** weist einen Trichter **700** auf. Der Trichter **700** ist ausgebildet und eingerichtet, einen zu behandelnden Bereich eines Patienten zu definieren, innerhalb dessen eine Flüssigkeit aus der Vorrichtung abgegeben werden kann.

In der hier gezeigten Ausführungsform sind der Adapter **200** und das Abgabeelement **300** als voneinander getrennte, und lösbar miteinander verbindbare, Teile ausgestaltet. Eine solche Ausgestaltung ermöglicht es, ein kontaminiertes Abgabeelement **300** während einer Operation durch ein neues, sauberes Abgabeelement **300** auszutauschen.

Der Behälter **100** weist ein Septum **103** auf, welches im hier gezeigten Beispiel bereits eine vorgeformte Durchführung aufweist, wobei diese Durchführung mit einem Siegel **112** verschlossen ist. Der Behälter **101** weist weiterhin ein erstes Verbindungselement **110** zur Verbindung mit dem Adapter **200** auf. Das erste Verbindungselement **110** ist hier als umlaufender Vorsprung ausgebildet. Der Adapter **200** weist ein zweites Verbindungselement **210** zur Verbindung mit dem ersten Verbindungselement **110** auf. Das zweite Verbindungselement **210** ist hier als flexibler Rasthaken ausgebildet. Der Adapter **200** weist weiterhin eine erste Durchführung **201** auf, welche sich durch einen Hohldorn **202** des Adapters **200** erstreckt. Der Hohldorn **202** ist zur Herstellung einer fluidleitenden Verbindung mit dem Behälter **101** ausgebildet und eingerichtet. Der Adapter weist eine stielförmige Düse **203** auf, die fluidleitend mit der ersten Durchführung **201** verbunden ist. Der Adapter **200** weist weiterhin ein drittes Verbindungselement **211** zur Verbindung mit dem Abgabeelement **300** auf. Das dritte Verbindungselement **211** ist hier als Außengewinde ausgebildet. Das Abgabeelement **300** weist ein viertes Verbindungselement **310** zur Verbindung mit dem Adapter **200** auf. Das vierte Verbindungselement **310** ist hier als Innengewinde ausgebildet. Das Abgabeelement **300** weist eine stielförmige Düse **303** mit einer zweiten Durchführung **301** und eine Austrittsöffnung **320** auf. Die Düse **203** des Adapters **200** ist ausgebildet und eingerichtet, eine formschlüssige Verbindung mit der Düse **303** des Abgabeelements **300** herzustellen. Hierdurch kann gleichzeitig die Herstellung einer fluidleitenden Verbindung zwischen der ersten Durchführung **201** des Adapters und der zweiten Durchführung **301** des Abgabeelements **300** erfolgen. Das Abgabeelement **300** weist einen Trichter mit einer Belüftungsöffnung **702** und eine Dichtlippe **703** auf. Die Dichtlippe **703** ist bevorzugt ausgebildet, mit einer Oberfläche eines Patientengewebes im Wesentlichen flüssigkeitsdicht abzuschließen. Das Abgabeelement **300** weist weiterhin ein fünftes Verbindungselement **311** zur Verbindung mit einem Zubehörteil **400** auf. Das fünfte Verbindungselement **311** ist hier als Schlauchanschluss ausgebildet, und das Zubehörteile **400** ist als Schlauch ausgebildet. Der Schlauch kann zum Absaugen von Flüssigkeit, die zuvor aus der Vorrichtung abgegeben und zum Reinigen eines Patientengewebes verwendet wurde, dienen. Wenn der Trichter mithilfe der Dichtlippe **703** bündig mit einer Oberfläche eines Patientengewebes abschließt, kann während eines Absaugvorgangs ein Druckausgleich über die Belüftungsöffnung **702** erfolgen. Wie hier gezeigt, ist es vorteilhaft, die Belüftungsöffnung **702** in einer Weise anzuordnen, dass die durch die Vorrichtung abgegebene Spülflüssigkeit die Belüftungsöffnung **702** bei bestimmungsgemäßem Gebrauch der Vorrichtung nicht erreicht. Hierzu kann es zweckmäßig sein, die Belüftungsöffnung **702** an einer Seite des Trichter **700** anzuordnen, welche einer Seite gegenüber liegt, an welcher die Dichtlippe **703** angeordnet ist. Weiterhin kann es vorteilhaft sein, die Belüftungsöffnung **702** und das fünfte Verbindungselement **311,** z.B. einen Schlauchanschluss, zueinander gegenüberliegend an dem Trichter **700** anzuordnen. Das fünfte Verbindungselement **311** kann hierzu in der Nähe der Dichtlippe **703** angeordnet sein. Falls der Trichter **700** keine Dichtlippe **703** aufweist, gelten diese Ausführungen für das entsprechende offene Ende des Trichters in entsprechender Weise.

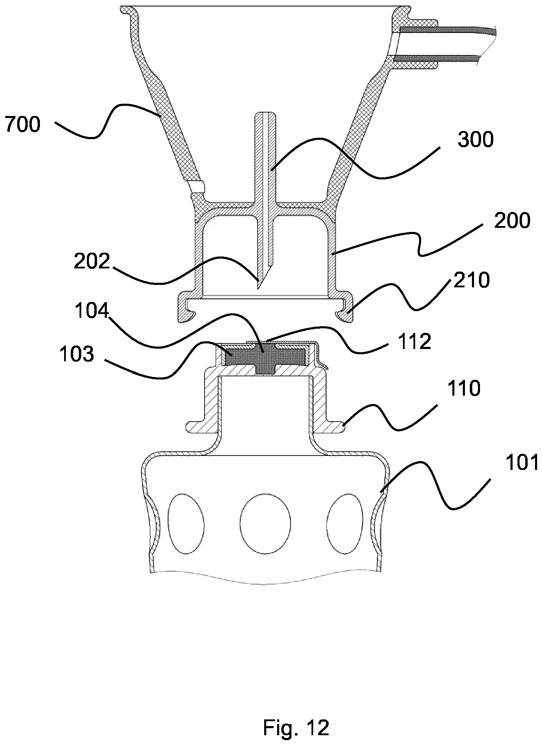

**Figur 12** zeigt einen Ausschnitt einer erfindungsgemäßen Vorrichtung **100** mit einem Behälter **101,** einem Adapter **200** und einem Abgabeelement **300,** wobei das Abgabeelement **300** einen Trichter **700** aufweist, in einer Querschnittsansicht. In der hier gezeigten Ausführungsform sind der Adapter **200** und das Abgabeelement **300** als miteinander fest verbundene Teile ausgebildet.

Der Behälter **101** weist ein erstes Verbindungselement **110** zur Verbindung mit dem Adapter **200** auf. Der Adapter **200** weist ein zweites Verbindungselement **210** zur Verbindung mit dem Behälter auf. In der hier gezeigten Ausführungsform sind ein drittes Verbindungselement und ein viertes Verbindungselement entbehrlich und nicht vorhanden, da der Adapter **200** untrennbar mit dem Abgabeelement **300** verbunden ist. Der Adapter **200** weist einen Hohldorn **202** zum Einführen in ein Septum **103** des Behälters **101** auf. Anders als bei der in Figur 11 gezeigten Ausführungsform handelt es sich in der hier gezeigten Ausführungsform um ein geschlossenes Septum **103,** welches durch den Hohldorn **202** durchstochen werden kann. Hierzu weist der Behälter **101** eine erste Durchstichposition **104** auf, welche durch eine oder mehrere Gehäuseöffnungen des Behälters **101** definiert ist. Beispielsweise kann der Behälter einen fest verbundenen Aufsatz aufweisen, welcher eine Halterung für das Septum **103,** eine Öffnung zum Durchstechen mit dem Hohldorn **202** und das erste Verbindungselement **110** aufweist.

Der Adapter **200** ist unmittelbar mit einem Abgabeelement **300** verbunden, welches einen Trichter **700** aufweist. In der hier gezeigten Ausführungsform ist der Hohldorn **202** des Adapters in das Abgabeelement **300** integriert.

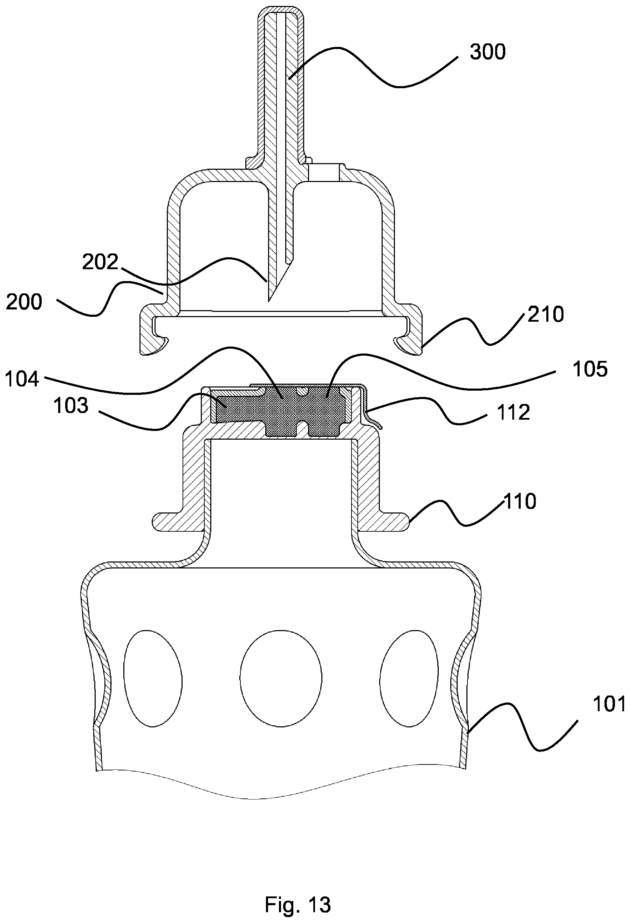

**Figur 13** zeigt einen Ausschnitt einer erfindungsgemäßen Vorrichtung **100** mit einem Behälter **101,** einem Adapter **200** und einem Abgabeelement **300** in einer Querschnittsansicht. In der hier gezeigten Ausführungsform sind der Adapter **200** und das Abgabeelement **300** als miteinander fest verbundene Teile ausgebildet. Der Behälter **101** weist einen fest verbundenen Aufsatz auf, welcher eine Halterung für das Septum **103,** und zwei Öffnungen aufweist, wobei diese beiden Öffnungen eine erste Durchstichposition **104** und eine zweite Durchstichposition **105** definieren. Dies ermöglicht es, den Behälter **101** gleichzeitig mit dem Adapter **200,** dem Abgabeelement **300** und einem Zubehörteil **400** (in dieser Figur nicht dargestellt) fluidleitend zu verbinden. Hierzu kann der Adapter **200** oder das Abgabeelement **300** eine entsprechende Öffnung aufweisen, sodass die zweite Durchstichposition **105** des Behälters **101** auch dann zugänglich bleibt, wenn der Behälter **101** mit dem Adapter **200** und gegebenenfalls dem Abgabeelement **300** verbunden ist. Somit kann in der ersten Durchstichposition **104** der Adapter **200** mithilfe des Hohldorns **202** fluidleitend mit dem Behälter **101** verbunden werden, und an der zweiten Durchstichposition **105** kann zusätzlich ein Zubehörteil **400** fluidleitend mit dem Behälter **101** verbunden werden. Das Zubehörteil **400** kann hierzu einen Hohldorn aufweisen, ähnlich wie der Adapter **200.**

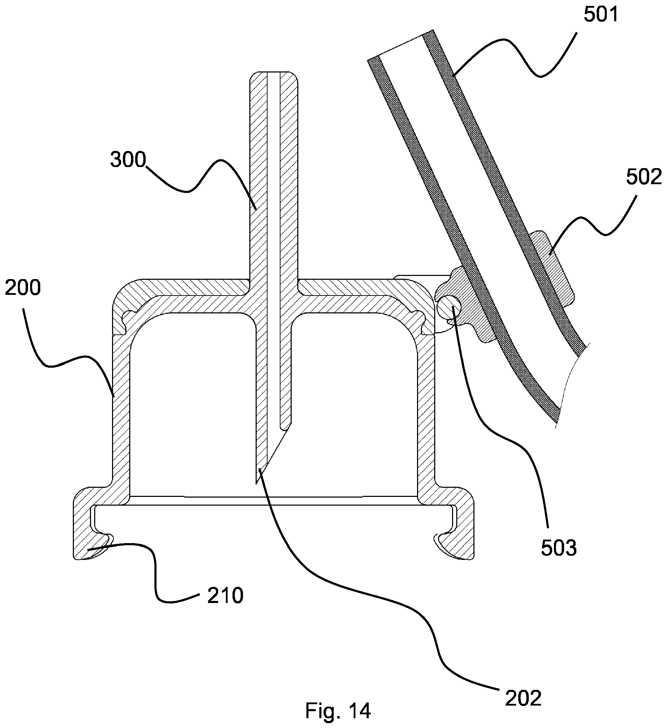

**Figur 14** zeigt einen Ausschnitt einer erfindungsgemäßen Vorrichtung **100** mit einem Adapter **200** und einem Abgabeelement **300** in einer Querschnittsansicht. In der hier gezeigten Ausführungsform sind der Adapter **200** und das Abgabeelement **300** als miteinander fest verbundene Teile ausgebildet. Der Adapter **200** weist ein zweites Verbindungselement **210** zur Verbindung mit dem Behälter **101** auf. Der Adapter **200** weist weiterhin einen Hohldorn **202** auf. Der Adapter **200** ist fest mit einem Abgabeelement **300** verbunden, und das Abgabeelement **300** ist in den Adapter **200** integriert. Das Abgabeelement **300** weist weiterhin ein Gelenk **503** zur beweglichen Verbindung mit einer Schelle **502** auf. Die Schelle umgibt und hält einen Schlauch **501.** Aufgrund des Gelenks **503** kann der Schlauch **501** relativ zu dem Abgabeelement **300** bewegt werden. Dies ermöglicht es einem Benutzer der Vorrichtung, gebrauchte Spülflüssigkeit von einer geeigneten Position eines Patientengewebes abzuführen.

**Figur 15** zeigt einen Ausschnitt einer erfindungsgemäßen Vorrichtung **100** mit einem Adapter **200** und einem Abgabeelement **300** in einer Querschnittsansicht. In der hier gezeigten Ausführungsform sind der Adapter **200** und das Abgabeelement **300** als miteinander fest verbundene Teile ausgebildet. Das Abgabeelement **300** weist einen Bürstenkopf **600** auf. Das Abgabeelement ist ausgebildet und eingerichtet, eine Flüssigkeit aus der Vorrichtung im Bereich des Bürstenkopfs **600** abzugeben. Das Abgabeelement **300** weist einen gekrümmten Bereich auf. Dies kann es einem Benutzer der Vorrichtung in bestimmten Behandlungssituationen ermöglichen, den Bürstenkopf **600** besser zu positionieren, als dies bei einem Abgabeelement **300** mit im Wesentlichen linearer Geometrie der Fall wäre.

**Figur 16** zeigt ein Abgabeelement **300** in einer Querschnittsansicht. Das Abgabeelement **300** weist einen Bürstenkopf **600** auf. Das Abgabeelement ist ausgebildet und eingerichtet, eine Flüssigkeit aus der Vorrichtung im Bereich des Bürstenkopfs **600** abzugeben. Das Abgabeelement **300** weist weiterhin ein Absaugelement **500** mit einem Schlauch **501** auf. Der Schlauch **501** ist mithilfe einer Schelle **502** an dem Abgabeelement **300** befestigt. Der Bürstenkopf **600** weist umlaufend angeordnete Borsten **601** auf. Im hier gezeigten Beispiel sind die Borsten **601** nach Art einer Rundbürste an dem Bürstenkopf **600** angeordnet. In dieser Ausführungsform erstreckt sich jeweils eine fluidleitende Durchführung durch einen Innenraum der Borsten **601,** welche jeweils in eine Austrittsöffnung **320** mündet. Somit ist die Vorrichtung ausgebildet und eingerichtet, eine Flüssigkeit durch die Borsten **601** hindurch an einen Patienten abzugeben.

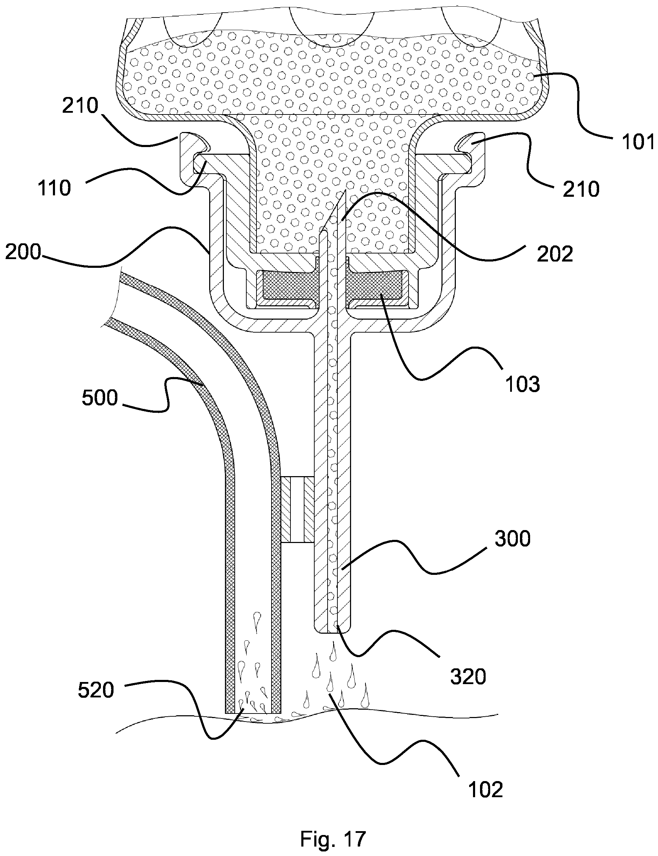

**Figur 17** zeigt eine Ausführungsform einer erfindungsgemäßen Vorrichtung, die zur Abgabe einer Flüssigkeit an einen Patienten und das gleichzeitige Abführen einer Flüssigkeit von dem Patienten ausgebildet und eingerichtet ist. Die Vorrichtung weist einen Behälter **101** auf, der über ein erstes Verbindungselement **110** und ein zweites Verbindungselement **210** mit einem Adapter **200** verbunden ist. In der hier gezeigten Ausführungsform sind der Adapter **200** und das Abgabeelement **300** als miteinander fest verbundene Teile ausgebildet. Der Adapter **200** weist einen Hohldorn **202** auf, der hier durch ein Septum **103** des Behälters **101** geführt ist. Die Vorrichtung weist weiterhin ein Abgabeelement **300** mit einer Austrittsöffnung **320** auf, aus der eine Flüssigkeit **102** abgegeben werden kann. Die Vorrichtung weist weiterhin ein Absaugelement **500** auf, das mit dem Abgabeelement **300** verbunden ist. Das Absaugelement **500** weist eine Absaugöffnung **520** zur Abführung einer Flüssigkeit auf.

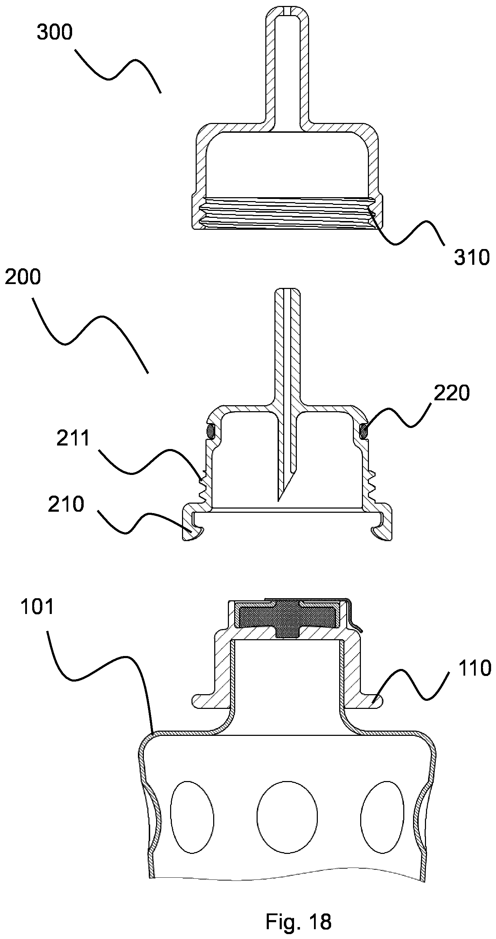

**Figur 18** zeigt eine Ausführungsform einer erfindungsgemäßen Vorrichtung, die eine zusätzliche Dichtung **220** aufweist. Die Vorrichtung weist einen Behälter **101** mit einem ersten Verbindungselement **110** auf. Ein als separates Teil ausgebildeter Adapter **200** weist ein zweites Verbindungselement **210** auf, das zur Verbindung mit dem ersten Verbindungselement **110** ausgebildet und eingerichtet ist. Der Adapter **200** weist eine umlaufende Rille auf, in der eine ringförmige Dichtung **220** nach Art eines "O-Rings" eingelegt ist. Hierdurch kann der Adapter **200** eine flüssigkeitsdichte Verbindung mit einem Abgabeelement **300** ausbilden. Der Adapter ist mithilfe eines Gewindes mit dem Abgabeelement **300** verbindbar. Eine Gewindeverbindung kann durch ein drittes Verbindungselement **211** am Adapter **200** und ein viertes Verbindungselement **310** am Abgabeelement **300** gebildet werden. Aufgrund der dichtenden Wirkung der Dichtung **220** kann die Dichtigkeit der druckfesten fluidleitenden Verbindung zwischen dem Adapter **200** und dem Abgabeelement **300,** die durch diese Gewindeverbindung erzielt wird, weiter verbessert werden.

### BEISPIEL 1

Eine handelsübliche Flasche mit steriler Kochsalzlösung wurde mithilfe eines hierin beschriebenen kappenförmigen Adapters und eines hierin beschriebenen kappenförmigen Abgabeelements verbunden.

Hierbei wurden die folgenden zwei Varianten miteinander verglichen:
In Variante A wurde die Verbindung zwischen der Flasche und dem kappenförmigen Adapter mithilfe eines einfachen Formschlusses hergestellt, welcher allein auf der korrespondierenden Innenform der Adapterkappe und der Außenform des Flaschenkopfs beruhte. In gleicher Weise wurde eine Verbindung zwischen dem Adapter und dem kappenförmigen Abgabeelement mithilfe eines einfachen Formschluss hergestellt, welcher auf der korrespondierenden Außenform der Adapterkappe und der Innenform des kappenförmigen Abgabeelements beruhte.

Die Adapterkappe war mit einer stielförmige Spritztülle ausgestattet, welche in eine ähnliche geformte Spritztülle des Abgabeelements eingeschoben wurde.

Durch manuellen Druck auf die Flasche wurde in mehreren Tests versucht, die in der Flasche befindliche Flüssigkeit über die Adapterkappe und das Abgabeelement abzugeben. Jedoch erwiesen sich die Verbindung zwischen Flasche und Adapterkappe und die Verbindung zwischen Adapterkappe und Abgabeelement als undicht, und in einigen Fällen löste sich die Adapterkappe von der Flasche oder dem Abgabeelement.

Variante B war im Wesentlichen mit Variante A identisch, wobei in Variante B die Adapterkappe mithilfe eines elastischen Rasthakens an einem Vorsprung des Flaschenkopfs befestigt wurde. Weiterhin wurde in Variante B die Adapterkappe mithilfe eines Gewindes mit dem Abgabeelement verbunden. Hierbei zeigte sich, dass bei manuellem Druck auf die Flasche keine Flüssigkeit zwischen Adapterkappe und Flasche oder zwischen Adapterkappe und Abgabeelement austrat, und die Adapterkabel fest mit der Flasche und dem Abgabeelement verbunden blieb. Variante B entsprach im Wesentlichen der Vorrichtung, welche in Figur 3 dargestellt ist.

Hierdurch wurde festgestellt, dass durch Verwendung der genannten Verbindungselemente zwischen Behälter, Adapter und Abgabeelement eine druckfeste fluidleitende Verbindung zwischen diesen drei Teilen hergestellt werden konnte.

### LISTE DER BEZUGSZEICHEN

- 100: Vorrichtung
- 101: Behälter
- 102: Flüssigkeit
- 103: Septum
- 104: erste Durchstichposition
- 105: zweite Durchstichposition
- 110: erstes Verbindungselement
- 112: Siegel
- 200: Adapter
- 201: erste Durchführung
- 202: Hohldorn
- 203: Düse
- 210: zweites Verbindungselement
- 211: drittes Verbindungselement
- 220: Dichtung
- 300: Abgabeelement
- 301: zweite Durchführung
- 303: Düse
- 310: viertes Verbindungselement
- 311: fünftes Verbindungselement
- 320: Austrittsöffnung
- 400: Zubehörteil
- 500: Absaugelement
- 501: Schlauch
- 502: Schelle
- 503: Gelenk
- 520: Absaugöffnung
- 600: Bürstenkopf
- 601: Borsten
- 602: Stielelement
- 700: Trichter
- 701: Gelenk
- 702: Belüftungsöffnung
- 703: Dichtlippe

## Patentansprüche

1. Vorrichtung (100) zur Abgabe einer medizinischen Flüssigkeit an einen Patienten, aufweisend
einen elastisch verformbaren Behälter (101) zur Aufnahme einer Flüssigkeit (102),
einen Adapter (200), der mit dem Behälter (101) fluidleitend verbindbar ist,
ein Abgabeelement (300) zur Abgabe einer Flüssigkeit (102) aus der Vorrichtung (100),
und ein Absaugelement (500) zur Abführung einer Flüssigkeit,
wobei die Vorrichtung (100) ausgebildet und eingerichtet ist, eine Flüssigkeit (102) durch Zusammendrücken des Behälters (101) mit einem Druck zu beaufschlagen, und hierdurch die Flüssigkeit (102) aus der Vorrichtung (100) an einen Patienten abzugeben, und gleichzeitig durch das Absaugelement (500) die abgegebene Flüssigkeit von dem Patienten abzuführen.

2. Vorrichtung nach Anspruch 1, wobei der Behälter (101), der Adapter (200) und das Abgabeelement (300) jeweils als miteinander lösbar verbindbare Teile ausgebildet sind.

3. Vorrichtung nach einem der vorangehenden Ansprüche, wobei das Absaugelement (500) einen Schlauch (501) oder ein Rohr aufweist.

4. Vorrichtung nach einem der vorangehenden Ansprüche, wobei das Absaugelement (500) durch eine Schelle (502) gehalten ist, wobei die Schelle (502) bevorzugt drehbar gelagert an der Vorrichtung angeordnet ist.

5. Vorrichtung nach einem der vorangehenden Ansprüche, wobei das Absaugelement (500) einen flexiblen oder gekrümmten Leitungsbereich (503) aufweist.

6. Vorrichtung nach einem der vorangehenden Ansprüche, wobei das Absaugelement (500) ein Gelenk (504) aufweist, wobei das Gelenk (504) bevorzugt als Scharnier oder Kugelgelenk ausgebildet ist.

7. Vorrichtung nach einem der vorangehenden Ansprüche, wobei die Vorrichtung ausgebildet und eingerichtet ist, eine Flüssigkeit (102) an einer ersten Position abzugeben, und die abgegebene Flüssigkeit an einer zweiten Position aufzunehmen, wobei die zweite Position relativ zu der ersten Position veränderlich ist, ohne die gesamte Vorrichtung (100) zu bewegen.

8. Vorrichtung nach einem der vorangehenden Ansprüche,
wobei der Behälter (101) ein erstes Verbindungselement (110) aufweist,
wobei der Adapter (200) ein zweites Verbindungselement (210), ein drittes Verbindungselement (211), und eine erste Durchführung (201) zur Abgabe einer Flüssigkeit (102) aus dem Behälter (101) aufweist,
wobei das Abgabeelement (300) ein viertes Verbindungselement (310) und eine zweite Durchführung (301) zur Abgabe einer Flüssigkeit (102) aus der Vorrichtung (100) aufweist,
und wobei die Vorrichtung ausgebildet und eingerichtet ist, durch Eingreifen des ersten Verbindungselements (110) in das zweite Verbindungselement (210) und durch Eingreifen des dritten Verbindungselements (211) in das vierte Verbindungselement (310) eine druckfeste fluidleitende Verbindung zwischen dem Behälter (100), dem Adapter (200) und dem Abgabeelement (300) herzustellen.

9. Vorrichtung nach einem der vorangehenden Ansprüche, weiterhin aufweisend ein Septum (103), das den Behälter (101) flüssigkeitsdicht verschließt, wobei der Adapter (200) eine erste Durchführung (201) zur Abgabe einer Flüssigkeit (102) aus dem Behälter (101) aufweist, und wobei der Adapter (200) ausgebildet und eingerichtet ist, durch Inkontaktbringen des Adapters (200) mit dem Behälter (101) das Septum (103) zu durchdringen, um eine fluidleitende Verbindung zwischen dem Behälter (101) und der ersten Durchführung (201) herzustellen.

10. Vorrichtung nach einem der vorangehenden Ansprüche, wobei das Abgabeelement (300) weiterhin ein fünftes Verbindungselement (311) aufweist, welches unabhängig von dem dritten Verbindungselement und dem vierten Verbindungselement mit einem Zubehörteil (400) verbindbar ist.

11. Vorrichtung nach Anspruch 10, wobei das Zubehörteil (400) ausgewählt ist aus der Gruppe bestehend aus einem Schlauch, einem Spritzschutzschirm, einer Lavage-Vorrichtung und einer Reinigungsbürste.

12. Vorrichtung nach einem der vorangehenden Ansprüche, wobei der Behälter (101) mit einer sterilen Flüssigkeit (102) befüllt ist, wobei die Flüssigkeit bevorzugt einen antiseptischen Wirkstoff umfasst, der ausgewählt ist aus der Gruppe bestehend aus Chlorhexidinglukonat, Benzalkoniumchlorid, Octenidindihydrochlorid, einem Alkalihypochlorit, einem Erdalkalihypochlorit, einem Alkalichlorit; und/oder wobei die Flüssigkeit Natriumdodecylsulfat oder physiologische Kochsalzlösung umfasst.

13. Vorrichtung nach Anspruch 12, wobei die sterile Flüssigkeit eine wässrige Lösung umfasst, die ausgewählt ist aus der Gruppe bestehend aus
einer Lösung von Benzalkoniumchlorid mit einer Benzalkoniumchlorid-Konzentration von 50 bis 1500 ppm;
einer Lösung von Chlorhexidindiglukonat und Benzalkoniumchlorid, wobei die Konzentration an Chlorhexidindiglukonat 50 bis 600 ppm beträgt und die Konzentration an Benzalkoniumchlorid 50 bis 250 ppm beträgt;
einer Lösung von Octenidindihydrochlorid mit einer Konzentration von 50 bis 2000 ppm;
einer Lösung eines Alkalihypochlorits mit einer Konzentration an Hypochlorit von 50 bis 2000 ppm;
einer Lösung eines Erdalkalihypochlorits mit einer Konzentration an Hypochlorit 50 bis 2000 ppm;
einer Lösung eines Alkalichlorits mit einer Konzentration an Chlorit von 50 bis 2000 ppm,
einer Lösung von Natriumdodecylsulfat mit einer Konzentration an Natriumdodecylsulfat von 50 bis 2000 ppm; und
einer physiologischen Kochsalzlösung mit einer Konzentration an Natriumchlorid von 0,9 Gew.-%;
wobei die ppm-Angaben sich jeweils auf einen Masse/Masse-Anteil im Verhältnis der jeweils genannten gelösten Stoffe zur Gesamtmasse der wässrigen Lösung beziehen.

14. Vorrichtung nach einem der vorangehenden Ansprüche, wobei das Abgabeelement (300) eine Austrittsöffnung (320) aufweist, und das Absaugelement (500) eine Absaugöffnung (520) aufweist, wobei die Austrittsöffnung (320) eine Querschnittsfläche aufweist, die kleiner oder gleich groß als/wie die Querschnittsfläche der Absaugöffnung (520) ist.

15. Vorrichtung nach einem der vorangehenden Ansprüche, wobei die Vorrichtung als in einem versiegelten, sterilen Packmittel angeordneter Teilesatz ausgebildet ist, und den Behälter (101), den Adapter (200) und das Abgabeelement (300) als jeweils getrennt voneinander vorliegende Teile umfasst.
